# EUROPEAN PATENT APPLICATION

(11) **EP 4 733 762 A1**
(43) Date of publication of application: **29.04.2026**
(21) Application number: 24826009.3
(22) Date of filing: 21.06.2024
(51) Int. Cl.: G01N 33/50, C07K 14/445, C07K 16/28, C07K 17/00, C12Q 1/02, G01N 33/53, G01N 33/574

(54) **CAPTURING METHOD AND DETECTION METHOD FOR EXTRACELLULAR VESICLES DERIVED FROM CANCER CELLS, AND KIT**

(30) Priority: 22.06.2023 JP 2023102262; 12.04.2024 JP 2024064488
(71) Applicant: Toray Industries, Inc., Tokyo 103-8666 (JP); Kyoto University, Kyoto-shi, Kyoto 606-8501 (JP)
(72) Inventor: SUDO, Tetsuo, Kyoto-shi, Kyoto 606-8501 (JP); OKANO, Kiyoshi, Kyoto-shi, Kyoto 606-8501 (JP); SERIZAWA, Takashi, Kamakura-shi, Kanagawa 248-8555 (JP)
(74) Representative: Mewburn Ellis LLP
(86) International application number: PCT/JP2024/022556
(87) International publication number: WO 2024/262608

(57) **Abstract**

A technology is disclosed for capturing or detecting extracellular vesicles derived from all cancers regardless of cancer type. A method of capturing cancer cell-derived extracellular vesicles contained in a biological sample includes a step of contacting the biological sample with (A) a polypeptide comprising IDI-ID2a domains of VAR2CSA protein derived from *Plasmodium falciparum,* (B) a polypeptide having 90% or more sequence identity with amino acid sequence of the polypeptide of (A) above, and binding to cancer cell-derived extracellular vesicles, or (C) a part of the polypeptide of (A) or (B) above, which is a polypeptide binding to cancer cell-derived extracellular vesicles, to bind the polypeptide and the cancer cell-derived extracellular vesicles.

## Description

### TECHNICAL FIELD

The present invention relates to a method of capturing cancer cell-derived extracellular vesicles, a method of detecting cancer-derived extracellular vesicles, and a kit.

### BACKGROUND ART

The leading cause of death in Japan is malignant neoplasms (cancer). Since cancer is almost completely curable if detected at an early stage, development of a minimally invasive technology capable of detecting cancer at the earliest possible stage is desired as one of the important measures for cancer prevention.

As a minimally invasive technology for detecting cancer, liquid biopsy (body fluid biopsy) is known, which can detect cancer by measuring extracellular vesicles (hereinafter sometimes referred to as "EVs") using a body fluid as a specimen, without performing a conventional biopsy to collect tumor tissue.

Cancer-derived EVs are expected to be difficult to detect because they are overwhelmingly fewer in number compared to EVs isolated from living organs and tissues. However, it is considered that cancer cell-derived EVs can be detected by combining antibodies against proteins specifically expressed on EVs, such as CD9, CD63, and CD81, with antibodies against proteins expressed on cancer cells, so-called cancer markers.

However, it has been reported that proteins contained in EVs show significantly different expression patterns from the proteins of the cells from which the EVs are derived (Non-Patent Literature 1). As a specific example, results were shown in which the membrane surfaces of cells and EVs produced from said cells were biotin-labeled, collected with avidin-coated beads, and their proteins were analyzed by LC-MS/MS, indicating that the membrane protein compositions of cells and their EVs are markedly different (Non-Patent Literature 2).

Therefore, to detect cancer cell-derived EVs, cancer markers for cancer cells cannot be simply used; it is necessary to analyze the proteins of cancer cell-derived EVs and find the proteins that can serve as markers.

Furthermore, several methods of detecting cancer cell-derived extracellular vesicles using markers expressed on particular cancer cells are known.

For example, regarding colon cancer, it was shown that colon cancer-derived EVs can be detected with antibodies targeting CD9, an EV marker, and CD147, which is expressed on colon cancer-derived EVs (Patent Literature 1). In fact, by analyzing proteins contained in cancer cell EVs, CD147, which is characteristically expressed on colon cancer-derived EVs, has been found.

Furthermore, regarding bladder cancer, proteomic analysis of EVs derived from the urines of bladder cancer patients was performed, and they identified CD55, CD82, etc. It was shown that bladder cancer-derived EVs can be detected with antibodies targeting CD9, an EV marker, and CD55 or CD82, which are expressed on bladder cancer-derived EVs (Patent Literature 2).

Moreover, in a paper on pancreatic cancer EVs, proteomic analysis of serum-derived EVs from pancreatic cancer patients was performed, and they found GPRC5C and EPS8 as markers. It has been shown that the expression levels of these markers in cells and extracellular vesicles in various pancreatic cancer cell lines and non-cancerous pancreatic cells do not correlate (Non-Patent Literature 3). This suggests that cancer markers found through expression analysis in cells cannot be simply applied as is as cancer markers for EVs.

On the other hand, it was reported that a VAR2CSA protein derived from *Plasmodium falciparum* binds to chondroitin sulfate A on cancer cells (Patent Literature 3, Non-Patent Literature 4). This cancer cell-derived chondroitin sulfate is also referred to as oncofetal chondroitin sulfate or placental-like chondroitin sulfate. However, there is no finding that said chondroitin sulfate is expressed on the surface layer of EVs. Furthermore, a method for measuring this placental-like chondroitin sulfate by ELISA was shown (Non-Patent Literature 5). However, this technology is for the detection of soluble oncofetal chondroitin sulfate in cell lysates or body fluids, and not a measurement for EVs.

### PRIOR ART DOCUMENTS

### PATENT LITERATURE

| | |
|---|---|
| Patent Literature 1: | WO 2014/167969 |
| Patent Literature 2: | WO 2016/143805 |
| Patent Literature 3: | JP 2015-513524 A |

### NON-PATENT LITERATURE

| | |
|---|---|
| Non-Patent Literature 1: | Jeppesen, DK. et al., 2019, Cell 177, 428-445 |
| Non-Patent Literature 2: | Kirkemo, LL. et al. eLife 2022;0:e73982. |
| Non-Patent Literature 3: | Yoshioka, Y.et al., Cancer Science. 2022;113:3498-3509 |
| Non-Patent Literature 4: | Salanti, A. et al., Cancer Cell. 2015; 28(4): 500-514. |
| Non-Patent Literature 5: | Zhang, J. et al., Int J. Med. Sci. 2020; 17: 161-169. |

### SUMMARY OF INVENTION

### PROBLEM TO BE SOLVED BY THE INVENTION

Conventionally, to detect cancer cell-derived EVs, it was necessary to obtain EV-specific markers for each cancer type, and a technology to isolate or detect cancer-derived EVs compatible with all cancer types had not been developed. For this reason, for early diagnosis of cancer, it was necessary to perform tests using a large number of markers for each cancer type.

An objective of the present invention is to provide a technology for capturing or detecting extracellular vesicles derived from all cancers regardless of cancer type.

### MEANS FOR SOLVING THE PROBLEM

The present inventors, in order to solve the above-mentioned problem, conducted intensive research and developed a technology to capture and detect cancer-derived extracellular vesicles using a VAR2CSA polypeptide derived from *Plasmodium falciparum,* which has the potential of binding to chondroitin sulfate on the surface of cancer cells. The present invention has been completed based on these findings.

The present invention provides the following:
(1) A method of capturing cancer cell-derived extracellular vesicles contained in a biological sample, the method comprising a step of contacting the biological sample with any one polypeptide of (A) to (C) below to bind the polypeptide and the cancer cell-derived extracellular vesicles:
   (A) a polypeptide containing ID1-ID2a domains of VAR2CSA protein derived from *Plasmodium falciparum;*
   (B) a polypeptide having 90% or more sequence identity with amino acid sequence of the polypeptide of (A) above, and binding to cancer cell-derived extracellular vesicles;
   (C) a part of the polypeptide of (A) or (B) above, which is a polypeptide binding to cancer cell-derived extracellular vesicles.
(2) The method of (1), wherein the ID1-ID2a domains of the VAR2CSA protein has an amino acid sequence of SEQ ID NO: 1, SEQ ID NO: 9, SEQ ID NO: 12, SEQ ID NO: 13, or SEQ ID NO: 14.
(3) The method of (1) or (2), wherein the polypeptide of (A) to (C) is immobilized on a support.
(4) The method of (1) or (2), wherein the biological sample is a cell culture supernatant, blood, serum, or plasma of a human.
(5) A method of detecting cancer cell-derived extracellular vesicles contained in a biological sample, the method comprising: a step of contacting the biological sample with any one polypeptide of (A) to (C) below to bind the polypeptide and the cancer cell-derived extracellular vesicles; and a step of detecting the cancer cell-derived extracellular vesicles bound to any one polypeptide of (A) to (C):
   (A) a polypeptide containing ID1-ID2a domain of a VAR2CSA protein derived from *Plasmodium falciparum;*
   (B) a polypeptide having 90% or more sequence identity with amino acid sequence of the polypeptide of (A) above, and binding to cancer cell-derived extracellular vesicles;
   (C) a part of the polypeptide of (A) or (B) above, which is a polypeptide binding to cancer cell-derived extracellular vesicles.
(6) The method of (5), further comprising contacting the cancer cell-derived extracellular vesicles with a second binding substance that binds to a surface of the extracellular vesicles, and comprising detecting the cancer cell-derived extracellular vesicles bound to the second binding substance and any one polypeptide of (A) to (C).
(7) The method of (6), wherein: either one of (i) any one polypeptide of (A) to (C) and (ii) the second binding substance is immobilized on a support, and the other is labeled; and the detection is performed by detecting the label.
(8) The method of (7), wherein the second binding substance is an antibody or an antigen-binding fragment thereof, or a phosphatidylserine-binding domain of Tim1, Tim3, Tim4, or beta-2-glycoprotein 1 (β2GP1), which is a protein that binds to phosphatidylserine located on outer side of membrane of the extracellular vesicles.
(9) The method of (8), wherein the antibody is an antibody against CD9, CD63, or CD81.
(10) The method of (6), wherein the biological sample is cell culture supernatant, blood, serum, or plasma of a human.
(11) The method of any one of (5) to (10), wherein the ID1-ID2a domains of the VAR2CSA protein has an amino acid sequence of SEQ ID NO: 1, SEQ ID NO: 9, SEQ ID NO: 12, SEQ ID NO: 13, or SEQ ID NO: 14.
(12) A method of obtaining information on whether or not a subject has cancer, the method being performed by detecting cancer cell-derived extracellular vesicles contained in a biological sample obtained from the subject by the method of any one of (1) to (11).
(13) A kit comprising: any one polypeptide of (A) to (C) below; and a second binding substance that binds to a surface of extracellular vesicles, wherein either one of (i) any one polypeptide of (A) to (C) and (ii) the second binding substance is immobilized on a support, and the other is labeled:
   (A) a polypeptide comprising ID1-ID2a domains of VAR2CSA protein derived from *Plasmodium falciparum;*
   (B) a polypeptide having 90% or more sequence identity with amino acid sequence of the polypeptide of (A) above, and binding to cancer cell-derived extracellular vesicles;
   (C) a part of the polypeptide of (A) or (B) above, which is a polypeptide binding to cancer cell-derived extracellular vesicles.
(14) The kit of (13), wherein the kit is for diagnosing whether or not a subject has cancer.

### EFFECT OF THE INVENTION

According to the present invention, cancer-derived extracellular vesicles can be captured and detected. Furthermore, a method of diagnosing cancer and a diagnostic kit based on the detection results of the cancer-derived extracellular vesicles are provided.

### BRIEF DESCRIPTION OF DRAWINGS

FIG. 1 is a diagram showing the structure of a polypeptide containing the ID1-ID2a domains of VAR2CSA protein used in Examples of the present invention.
FIG. 2 is a schematic diagram illustrating an example of the analysis method of the present invention.
FIG. 3 is a figure showing results of detecting purified EVs derived from U87-MG cells, captured with a VAR2CSA, using an anti-CD63 antibody.
FIG. 4 is a figure showing results of detecting purified EVs derived from U87-MG cells, captured with an anti-CD9 antibody, using an anti-CD63 antibody.
FIG. 5 is a figure showing results of detecting purified EVs derived from WI-38-hTERT cells, captured with a VAR2CSA, using an anti-CD63 antibody.
FIG. 6 is a figure showing results of detecting purified EVs derived from WI-38-hTERT cells, captured with an anti-CD9 antibody, using an anti-CD63 antibody.
FIG. 7 is a figure showing results of detecting purified EVs derived from pancreatic cancer cell lines KMP2 and PANC-1 cells, captured with a VAR2CSA, using a mixed antibody of an anti-CD9 antibody and an anti-CD81 antibody.
FIG. 8 is a figure showing results of detecting purified EVs derived from pancreatic cancer cell lines KMP2 and PANC-1 cells, captured with Tim4, using a mixed antibody of an anti-CD9 antibody and an anti-CD81 antibody.
FIG. 9 is a figure showing results of detecting purified EVs derived from prostate cancer cell line PC-3 cells and prostate cancer cells LNCap cells, normal prostate epithelial cell line HPrEC-TKD cells, and normal prostate fibroblast cell line HPrF-TKD cells, captured with a VAR2CSA, using a mixed antibody of three types: an anti-CD9 antibody, an anti-CD63 antibody, and an anti-CD81 antibody.
FIG. 10 is a figure showing results of detecting purified EVs derived from prostate cancer cell line PC-3 cells and prostate cancer cells LNCap cells, normal prostate epithelial cell line HPrEC-TKD cells, and normal prostate fibroblast cell line HPrF-TKD cells, captured with Tim4, using a mixed antibody of three types: an anti-CD9 antibody, an anti-CD63 antibody, and an anti-CD81 antibody.
FIG. 11 is a figure showing results of detecting purified EVs derived from lung cancer cell lines H69AR3 cells, NCI-H1703 cells, and A549 cells, and normal fetal lung fibroblast cell line WI-38-hTERT cells, captured with a VAR2CSA, using a mixed antibody of three types: an anti-CD9 antibody. an anti-CD63 antibody, and an anti-CD81 antibody.
FIG. 12 is a figure showing results of detecting purified EVs derived from lung cancer cell lines H69AR3 cells, NCI-H1703 cells, and A549 cells, and normal fetal lung fibroblast cell line WI-38-hTERT cells, captured with Tim4, using a mixed antibody of three types: an anti-CD9 antibody, an anti-CD63 antibody, and an anti-CD81 antibody.
FIG. 13 is a figure showing results of detecting purified EVs derived from osteosarcoma cell line MG-63 cells and normal osteoblast cell line NHOst-TKD cells, captured with a VAR2CSA, using a mixed antibody of three types: an anti-CD9 antibody, an anti-CD63 antibody, and an anti-CD81 antibody.
FIG. 14 is a figure showing results of detecting purified EVs derived from osteosarcoma cell line MG-63 cells and normal osteoblast cell line NHOst-TKD cells, captured with Tim4, using a mixed antibody of three types: an anti-CD9 antibody, an anti-CD63 antibody, and an anti-CD81 antibody.
FIG. 15 is a figure showing results of detecting purified EVs derived from acute myeloid leukemia cell line KG-1 cells, captured with a VAR2CSA, using an anti-CD63 antibody. Furthermore, it is also a figure showing that the capture of the extracellular vesicles by a VAR2CSA is inhibited by chondroitin sulfate A (CSA).
FIG. 16 is a figure showing results of detecting purified EVs derived from acute myeloid leukemia cell line KG-1 cells, captured with a VAR2CSA or Tim4, using an anti-CD63 antibody.
FIG. 17 is a figure showing results of detecting purified EVs from esophageal cancer cell lines KYSE150 cells, KYSE510 cells, and KYSE1240 cells, captured with a VAR2CSA, using a mixed antibody of three types: an anti-CD9 antibody, an anti-CD63 antibody, and an anti-CD81 antibody.
FIG. 18 is a figure showing results of detecting purified EVs from esophageal cancer cell lines KYSE150 cells, KYSE510 cells, and KYSE1240 cells, captured with Tim4, using a mixed antibody of three types: an anti-CD9 antibody, an anti-CD63 antibody, and an anti-CD81 antibody.
FIG. 19 is a figure showing results of detecting purified EVs from stomach cancer cell lines IIGC-27 cells, AGS cells, and MKN45 cells, captured with a VAR2CSA, using a mixed antibody of three types: an anti-CD9 antibody, an anti-CD63 antibody, and an anti-CD81 antibody.
FIG. 20 is a figure showing results of detecting purified EVs from stomach cancer cell lines HGC-27 cells, AGS cells, and MKN45 cells, captured with Tim4, using a mixed antibody of three types: an anti-CD9 antibody, an anti-CD63 antibody, and an anti-CD81 antibody.
FIG. 21 is a figure showing results of detecting purified EVs from colon cancer cell lines SW837 cells, SW480 cells, HTC-116 cells, and Caco-2 cells, captured with a VAR2CSA, using a mixed antibody of three types: an anti-CD9 antibody, an anti-CD63 antibody, and an anti-CD81 antibody.
FIG. 22 is a figure showing results of detecting purified EVs from colon cancer cell lines SW837 cells, SW480 cells, HTC-116 cells, and Caco-2 cells, captured with Tim4, using a mixed antibody of three types: an anti-CD9 antibody, an anti-CD63 antibody, and an anti-CD81 antibody.
FIG. 23 is a figure showing results of detecting purified EVs from EpCAM-positive pancreatic cancer cell lines KMP2 and PANC-1, and EpCAM-negative pancreatic cancer cell line MIA Paca-2 cells, captured with a VAR2CSA polypeptide, using a mixed antibody of three types: an anti-CD9 antibody, an anti-CD63 antibody, and an anti-CD81 antibody.
FIG. 24 is a figure showing results of detecting purified EVs from EpCAM-positive pancreatic cancer cell lines KMP2 and PANC-1, and EpCAM-negative pancreatic cancer cell line MIA Paca-2 cells, captured with Tim4, using a mixed antibody of three types: an anti-CD9 antibody, an anti-CD63 antibody, and an anti-CD81 antibody.
FIG. 25 is a figure showing results of serially diluting cell culture supernatants of glioblastoma cell line U87-MG cells and normal fetal lung fibroblast cell line WI-38-hTERT cells, adding them to an anti-CD63 antibody-immobilized plate to capture EVs in the samples, and detecting the captured EVs with a VAR2CSA polypeptide.
FIG. 26 is a figure showing results of detecting purified EVs derived from lung cancer cell line NCI-H1703 cells and purified EVs derived from normal fetal lung fibroblast cell line WI-38-hTERT cells, added to bovine serum, captured with a VAR2CSA, using a mixed antibody of three types: an anti-CD9 antibody, an anti-CD63 antibody, and an anti-CD81 antibody.
FIG. 27 is a figure showing a comparison of EV capture efficiencies on differences in immobilization of a VAR2CSA polypeptide onto a support. The numerical values in the figure are the ratio of the value obtained with the nickel chelate-coated plate against the value obtained with the streptavidin-coated plate.
FIG. 28 is a figure showing results of SDS polyacrylamide gel electrophoresis (SDS-PAGE) of five types of purified VAR2CSA polypeptides.
FIG. 29 is a figure showing results of binding analysis (ELISA) of five types of VAR2CSA polypeptides to bovine decorin.
FIG. 30 is a figure showing results of binding analysis (ELISA) of five types of VAR2CSA polypeptides to EVs derived from leukemia-derived cell line KG-1 cells and normal fetal lung fibroblast cell line WI-38-hTERT cells.
FIG. 31 is a figure showing results of detecting purified EVs from kidney cancer cell lines Caki-1 cells and Caki-2 cells, and normal kidney epithelial cell line HREC-TKD cells, captured with a VAR2CSA, using a mixed antibody of three types: an anti-CD9 antibody, an anti-CD63 antibody, and an anti-CD81 antibody.
FIG. 32 is a figure showing results of detecting purified EVs from kidney cancer cell lines Caki-1 cells and Caki-2 cells, and normal kidney epithelial cell line HREC-TKD cells, captured with Tim4, using a mixed antibody of three types: an anti-CD9 antibody, an anti-CD63 antibody, and an anti-CD81 antibody.
FIG. 33 is a figure showing results of detecting purified EVs from breast cancer cell lines MDA-MB-231 cells, MDA-MB-45 cells, and T47D cells, and normal breast epithelial cell line HMEC-TKD cells, captured with a VAR2CSA, using a mixed antibody of three types: an anti-CD9 antibody, an anti-CD63 antibody, and an anti-CD81 antibody.
FIG. 34 is a figure showing results of detecting purified EVs from breast cancer cell lines MDA-MB-231 cells, MDA-MB-45 cells, and T47D cells, and normal breast epithelial cell line HMEC-TKD cells, captured with Tim4, using a mixed antibody of three types: an anti-CD9 antibody, an anti-CD63 antibody, and an anti-CD81 antibody.
FIG. 35 is a figure showing results of detecting purified EVs from ovarian cancer cell line OVCAR3 cells and bladder cancer cell line T24 cells, captured with a VAR2CSA, using a mixed antibody of three types: an anti-CD9 antibody, an anti-CD63 antibody, and an anti-CD81 antibody.
FIG. 36 is a figure showing results of detecting purified EVs from ovarian cancer cell line OVCAR3 cells and bladder cancer cell line T24 cells, captured with Tim4, using a mixed antibody of three types: an anti-CD9 antibody, an anti-CD63 antibody, and an anti-CD81 antibody.
FIG. 37 is a figure showing results of detecting purified EVs from liver cancer cell line HepG2 cells, gallbladder cancer cell line TYGBK-8 cells, and bile duct cancer cell lines HuCCT1 cells and TFK-1 cells, captured with a VAR2CSA, using a mixed antibody of three types: an anti-CD9 antibody, an anti-CD63 antibody, and an anti-CD81 antibody.
FIG. 38 is a figure showing results of detecting purified EVs from liver cancer cell line HepG2 cells, gallbladder cancer cell line TYGBK-8 cells, and bile duct cancer cell lines HuCCT1 cells and TFK-1 cells, captured with Tim4, using a mixed antibody of three types: an anti-CD9 antibody, an anti-CD63 antibody, and an anti-CD81 antibody.
FIG. 39 is a figure showing results of detecting purified EVs from acute lymphocytic leukemia cell line NALM-6 cells and acute myeloid leukemia cell line KG-1 cells, captured with a VAR2CSA, using a mixed antibody of three types: an anti-CD9 antibody, an anti-CD63 antibody, and an anti-CD81 antibody.
FIG. 40 is a figure showing results of detecting purified EVs from acute lymphocytic leukemia cell line NALM-6 cells and acute myeloid leukemia cell line KG-1 cells, captured with Tim4, using a mixed antibody of three types: an anti-CD9 antibody, an anti-CD63 antibody, and an anti-CD81 antibody.
FIG. 41 is a figure showing results of detecting purified EVs from tracheal smooth muscle cell line HBSMC-TKD cells, aortic endothelial cell line HAEC-TKD cells, and acute myeloid leukemia cell line KG-1 cells, captured with a VAR2CSA, using a mixed antibody of three types: an anti-CD9 antibody, an anti-CD63 antibody, and an anti-CD81 antibody.
FIG. 42 is a figure showing results of detecting purified EVs from tracheal smooth muscle cell line HBSMC-TKD cells and aortic endothelial cell line HAEC-TKD cells, captured with Tim4, using a mixed antibody of three types: an anti-CD9 antibody, an anti-CD63 antibody, and an anti-CD81 antibody.
FIG. 43 is a figure showing the procedure for purifying EVs from human healthy control plasma spiked with extracellular vesicles derived from acute myeloid leukemia cell line KG-1 cells.
FIG. 44 is a figure showing results of detecting purified EVs from healthy control plasma spiked with the extracellular vesicles derived from acute myeloid leukemia cell line KG-1 cells, captured with a VAR2CSA, using a mixed antibody of three types: an anti-CD9 antibody, an anti-CD63 antibody, and an anti-CD81 antibody.
FIG. 45 is a figure showing results of detecting purified EVs from healthy control plasma spiked with the extracellular vesicles derived from acute myeloid leukemia cell line KG-1 cells, captured with Tim4, using a mixed antibody of three types: an anti-CD9 antibody, an anti-CD63 antibody, and an anti-CD81 antibody.
FIG. 46 is a figure showing results of detecting purified EVs from colon cancer patient plasma and healthy control plasma, as well as purified EVs from leukemia cell line KG-1 cells and colon cancer cell line HTC-116 cells as controls, and buffer as a control, captured with a VAR2CSA, using a mixed antibody of three types: an anti-CD9 antibody, an anti-CD63 antibody, and an anti-CD81 antibody.
FIG. 47 is a figure showing results of detecting purified EVs from colon cancer patient plasma and healthy control plasma, as well as purified EVs from leukemia cell line KG-1 cells and colon cancer cell line HTC-116 cells as controls, and buffer as a control, captured with Tim4, using a mixed antibody of three types: an anti-CD9 antibody, an anti-CD63 antibody, and an anti-CD81 antibody.

### MODE FOR CARRYING OUT THE INVENTION

Hereinafter, embodiments of the present invention will be described in detail. Note that the present invention can be carried out using conventional techniques in molecular biology and immunology that are within the skill of the art in this field. Such techniques are fully described in literature.

The present invention is a method of capturing cancer cell-derived extracellular vesicles contained in a biological sample, the method comprising a step of contacting the biological sample with (A): a polypeptide containing ID1-ID2a domains of VAR2CSA protein derived from *Plasmodium falciparum;* (B): a polypeptide having 90% or more sequence identity with the amino acid sequence of the polypeptide of (A) above, and binding to cancer cell-derived extracellular vesicles; or (C): a part of the polypeptide of (A) or (B) above, which is a polypeptide binding to cancer cell-derived extracellular vesicles, to bind the polypeptide and the cancer cell-derived extracellular vesicles.

### 1. Extracellular Vesicles

Extracellular vesicles (hereinafter sometimes referred to as EVs) are defined as particles released from cells, surrounded by a lipid bilayer membrane, and lacking a nucleus. They are classified into exosomes, microvesicles, and apoptotic bodies based on differences in production mechanisms.

Exosomes are vesicles derived from endosomal membranes formed during the endocytic process, with a size of about 30-200 nm. Microvesicles are considered vesicles of a wide range of sizes, 100-1000 nm, and differ from exosomes in that they are secreted directly from the cell membrane by budding. Apoptotic bodies are particles with a size of 50-5000 nm that are budded from the membrane of apoptotic cells, similar to microvesicles.

Furthermore, extracellular vesicles may contain proteins, RNA (mRNA, miRNA, non-coding RNA), DNA fragments, and the like.

### 2. Plasmodium falciparum VAR2CSA polypeptide

In the method of the present invention, *a Plasmodium falciparum* VAR2CSA polypeptide or a polypeptide derived therefrom is used. Note that the finding, specifically described in the Examples below, that the VAR2CSA polypeptide binds to cancer cell-derived extracellular vesicles but does not bind to normal cell-derived extracellular vesicles, is a new finding discovered by the present invention. Hereinafter, first, the *Plasmodium falciparum* VAR2CSA polypeptide will be described.

In pregnant women infected with *Plasmodium falciparum,* it is known that infected red blood cells accumulate in the placental villi, impairing placental function, leading to miscarriage or stillbirth of the fetus. The binding of malaria parasite-infected red blood cells to the placenta involves the binding of a protein belonging to the members of *Plasmodium falciparum* erythrocyte membrane protein 1 (PFEMP1), expressed on malaria parasite-infected red blood cells, to chondroitin sulfate proteoglycans of the placenta. PFEMP1 of the malaria parasite is encoded by about 60 types of var genes, and among them, the var2csa gene has been shown to be responsible for binding to chondroitin sulfate proteoglycans of the placenta (Baruch, D. I. et al., Cell, 1995; 82, 77-87, Kraemer, S. M. & Smith, J. D., Curr. Opin. Microbiol. 2006; 9, 374-380).

Furthermore, it has been shown that the VAR2CSA polypeptide encoded by the var2csa gene specifically binds to glycosaminoglycans consisting of chondroitin sulfate A (Salanti, A. et al., Mol. Microbiol. 2003; 49, 179-191), and that the VAR2CSA polypeptide binds to chondroitin sulfate A of various cancer cell lines (Salanti, A. et al., Cancer Cell 2015; 28, 500-514).

A plurality of strains of *Plasmodium falciparum* exist, and specifically, FCR3, K1, 3D7, Dd2, T9/94, Tak9/96, MAD20, K39, Honduras-1, HB3, 7G8, FCB, V1/S, M0920 and M.Camp strains are known. The FCR3, K1, 3D7, Dd2, T9/94, Tak9/96, MAD20, K39, Honduras-1, HB3, 7G8, FCB and V1/S strains are preserved in the Biological Resource Room, Institute of Tropical Medicine, Nagasaki University, and are available.

Regarding the base sequence information of the var2csa gene and the corresponding amino acid sequence information of isolates separated from patients infected with *Plasmodium falciparum,* FCR3 is GenBank (tradename) accession no. GU249598 and AAQ73926, 3D7 is JQ247428, NF54 is XM001350379, HB3 is KOB63403, 7G8 is EUR69480, M.Camp is OK346631, M0920 is OK346630, M200101 is QGY73032, WR80 is ABS79817, and Dd2 is KOB85905, which are available from databases of nucleic acid base sequences and protein amino acid sequences such as GenBank.

The homology among strains of *Plasmodium falciparum* of the VAR2CSA polypeptide of isolates separated from patients infected with *Plasmodium falciparum* is 70-80% on average, but infection in humans occurs with all strains, and symptoms such as placental dysfunction, miscarriage, and stillbirth are observed. Based on the infection mechanism described above, it is considered that the VAR2CSA polypeptide of each strain possesses binding properties to chondroitin sulfate A. The VAR2CSA polypeptide of the present invention is preferably a VAR2CSA polypeptide of an isolate separated from a patient infected with *Plasmodium falciparum.* Therefore, in the VAR2CSA polypeptide, it is considered to retain the extracellular vesicle capturing ability shown in the Examples of the present invention, as long as it is the original amino acid sequence among the *Plasmodium falciparum* strains and no mutations are introduced into arbitrary amino acids. On the other hand, in the VAR2CSA polypeptides of those isolates separated from patients infected with *Plasmodium falciparum,* it is also possible to introduce insertions, deletions, or mutations into arbitrary amino acids, as long as the binding property to chondroitin sulfate A is not lost.

VAR2CSA is a 350 kD protein composed of six Duffy-Binding Like (DBL) domains and several inter-domain regions (ID). Furthermore, ID2a and ID2b of the IDs are also called Cysteine-rich Inter-Domain Regions (CIDR).

In binding tests of each domain with chondroitin sulfate A, it has been shown that polypeptides consisting of DBL1X-ID1-DBL2X-ID2a-ID2h (D13L1X-ID2b), DBL1X-ID1-DBL2X-ID2a (DBL1X-ID2a), and ID1-DBL2X-ID2a (ID1-ID2a) bind to chondroitin sulfate proteoglycans of decorin derived from bovine articular cartilage (Clausen, T. M. et al., J. Biol. Chem. 2012; 287, 23332-23345). Furthermore, regarding the capture of cancer cells by the VAR2CSA polypeptide, it has been shown in comparative experiments between DBL1X-ID2a and ID1-ID2a that both have equivalent effects (Sand, N. T., Int. J. Mol. Sci. 2020; 21, 2401-2416).

In the present invention, as the VAR2CSA polypeptide, an ID1- ID2a polypeptide fragment derived from *Plasmodium falciparum* FCR3 strain, 7G8 strain, M0920 strain, 3D7 strain or M.Camp strain can be preferably used. The amino acid sequences of said polypeptides are shown in SEQ ID NO: 1, SEQ ID NO: 9, SEQ ID NO: 12, SEQ ID NO: 13, and SEQ ID NO: 14, respectively, in the Sequence Listing. Furthermore, a polynucleotide sequence encoding the polypeptide of SEQ ID NO: 1 is shown in SEQ ID NO: 2, and a polynucleotide sequence encoding the polypeptide of SEQ ID NO: 9 is shown in SEQ ID NO: 10. These polypeptide sequences are described in GenBank as accession numbers GU249598, EUR69480, OK346630, JQ247428, and OK346631, respectively.

In the method of the present invention.
(A) a polypeptide containing ID1-ID2a domains of VAR2CSA protein derived from *Plasmodium falciparum;*
(B) a polypeptide having 90% or more sequence identity with the amino acid sequence of the polypeptide of (A) above, and binding to cancer cell-derived extracellular vesicles; or
(C) a part of the polypeptide of (A) or (B) above, which is a polypeptide binding to cancer cell-derived extracellular vesicles
is used.

As the VAR2CSA protein, the full length can be used, but since the part that binds to oncofetal chondroitin sulfate A present on the surface of cancer cell-derived extracellular vesicles is the ID1-ID2a domains of the VAR2CSA protein, a polypeptide consisting of the ID1-ID2a domains can be used. Furthermore, a polypeptide containing this ID1-ID2a domains (e.g., the full length of the VAR2CSA protein, etc.) also binds to oncofetal chondroitin sulfate A, and thus can be used in the method of the present invention.

A polypeptide having 90% or more, preferably 95% or more, more preferably 97% or more, still more preferably 99% or more sequence identity with the amino acid sequence of the polypeptide of (A) above, and binding to cancer cell-derived extracellular vesicles can also be used in the method of the present invention. Here, the sequence identity of amino acid sequences is a value expressed as a percentage, which is obtained by aligning two amino acid sequences so that the number of matching amino acid residues is maximized (inserting gaps if necessary), and dividing the number of matched amino acid residues by the total number of amino acid residues (or the number of amino acid residues of the shorter sequence if the total lengths of the two amino acid sequences are different). Sequence identity can be easily calculated by a person skilled in the art using FASTA or the like, which is available for free on the Internet. In the present invention, if the total number of amino acid residues is less than 20, sequence identity is not defined.

The polypeptide containing the ID1-ID2a domains of VAR2CSA protein derived from *Plasmodium falciparum* of the present invention is preferably a polypeptide containing the ID 1-ID2a domains of VAR2CSA protein derived from a *Plasmodium falciparum* isolate separated from a patient infected with *Plasmodium falciparum.* This is because, as described above, said amino acid sequence is considered to have binding properties to chondroitin sulfate A.

As the amino acid sequence of the ID1-ID2a domains of the VAR2CSA protein described above, multiple sequences are known, and those shown in Table 1 below can be enumerated.

**[Table 1-1]**

| SEQ ID NO: | Isolate name | GenBank No. | Number of amino acid residues | FCR3 identity (%) |
|---|---|---|---|---|
| 1 | FCR3 | GU249598 | 640 | 100 |
| 9 | 7G8 | EUR69480 | 632 | 81 |
| 12 | M0920 | OK346630 | 620 | 65 |
| 13 | 3D7 | JQ247428 | 632 | 80 |
| 15 | NF54 | XM001350379 | 632 | 80 |
| 16 | HB3 | KOB63403 | 643 | 80 |
| 14 | M.Camp | OK346631 | 631 | 74 |
| 17 | M200101 | QGY73032 | 642 | 78 |
| 18 | Dd2 | KOB85905 | 628 | 77 |
| 19 | VIS | ABS79813 | 628 | 81 |
| 20 | T2C6 | ABS79814 | 637 | 75 |
| 21 | Indo | ABS79815 | 639 | 73 |
| 22 | MTS1 | ABS79816 | 646 | 76 |
| 23 | WR80 | ABS79817 | 642 | 64 |
| 24 | 124-8 | ABS79818 | 640 | 76 |
| 25 | KMWII | ABS79819 | 643 | 79 |
| 26 | M24 | ABS79820 | 656 | 79 |
| 27 | P13 | ABS79821 | 647 | 73 |
| 28 | PC49 | ABS79822 | 632 | 81 |
| 29 | FCH/4 | ETW30217 | 632 | 81 |
| 30 | Palo Alto/Uganda | ETW56275 | 649 | 89 |
| 31 | NF135/5.C10 | ETW41644 | 644 | 73 |
| 32 | MaliPS096_E11 | ETW46191 | 672 | 69 |
| 33 | UGT5.1 | EWC75615 | 655 | 72 |
| 34 | Santa Lucia | EUT82883 | 630 | 81 |
| 35 | RAJ116 | KNC35427 | 653 | 76 |
| 36 | PS103 | QGY73033 | 662 | 76 |
| 37 | PS122 | QGY73034 | 590 | 65 |
| 38 | PPRFG01_00_30 | SCM18829 | 643 | 66 |
| 39 | OPT79_1 | ALB01037 | 666 | 79 |
| 40 | OPT79_2 | ALB01038 | 611 | 73 |
| 41 | OPT79_3 | ALB01039 | 655 | 71 |
| 42 | OPT79_4 | ALB01040 | 658 | 75 |
| 43 | OPT80_1 | ALB01041 | 656 | 71 |
| 44 | OPT80_2 | ALB01042 | 658 | 73 |
| 45 | OPT80_3 | ALB01043 | 658 | 75 |
| 46 | OPT80_4 | ALB01044 | 633 | 80 |
| 47 | OPT90_1 | ALB01045 | 648 | 59 |
| 48 | OPT90_2 | ALB01046 | 670 | 70 |
| 49 | OPT112 | ALB01047 | 630 | 61 |
| 50 | OPT124 | ALB01048 | 651 | 70 |

**[Table 1-2]**

| SEQ ID NO: | Isolate name | GenBank No. | Number of amino acid residues | FCR3 identity (%) |
|---|---|---|---|---|
| 51 | OPT127_1 | ALB01049 | 611 | 60 |
| 52 | OPT127_2 | ALB01050 | 633 | 80 |
| 53 | OPT127_3 | ALB01051 | 630 | 59 |
| 54 | OPT129_1 | ALB01053 | 633 | 80 |
| 55 | OPT132 | ALB01054 | 634 | 72 |
| 56 | OPT141 | ALB01055 | 651 | 71 |
| 57 | OPT144 | ALB01056 | 650 | 72 |
| 58 | OPT152 | ALB01057 | 653 | 75 |
| 59 | OPT161_1 | ALB01058 | 611 | 74 |
| 60 | OPT161_2 | ALB01059 | 623 | 61 |
| 61 | HB3-1 | KOB63857 | 650 | 77 |
| 62 | Ghanal | none | 652 | 76 |
| 63 | Ghana2 | none | 667 | 68 |

As described above, among these, those represented by FCR3 (SEQ ID NO: 1), 7G8 (SEQ ID NO: 9), M0920 (SEQ ID NO: 12), 3D7 (SEQ ID NO: 13), or M. Camp (SEQ ID NO: 14) are preferable, and FCR3 (SEQ ID NO: 1) is most preferable.

Furthermore, a part of the polypeptide of (A) or (B) above, which is a polypeptide binding to cancer cell-derived extracellular vesicles, can also be used in the method of the present invention. The number of amino acid residues of this partial polypeptide is not particularly limited as long as it binds to cancer cell-derived extracellular vesicles, but is usually 20 or more, preferably 30 or more, more preferably 40 or more, still more preferably 50 or more, and most preferably 100 or more.

"Binding" in the "polypeptide binding to cancer cell-derived extracellular vesicles" of the present invention may be any binding as long as cancer cell-derived extracellular vesicles can be detected by the method of detecting of the present invention.

A Tag peptide sequence can be fused to the N-terminus and/or C-terminus of any one polypeptide of (A) to (C) above used in the method of the present invention (hereinafter, referred to as "VAR2CSA polypeptide" unless otherwise specified and not evident from the context) for the purpose of purification or detection of said polypeptide.

As the Tag peptide, FLAG-Tag, 3xFLAG-Tag, HA-Tag, Myc-Tag, HIS-Tag, V5-Tag, VSV-G-Tag, CBD-Tag, CBP-Tag, GST-Tag, MBP-Tag, Thioredoxin-Tag, SNAP-Tag, CLIP-Tag, Strep-Tag II, Twin-Strep-tag, SpyTag, Avi-Tag, and the like can be suitably enumerated.

The polypeptide consisting of the domain of VAR2CSA that binds to chondroitin sulfate proteoglycans can be produced in *E. coli* or insect cells, but is not limited thereto, and can also be produced in animal cells such as 293 cells. Furthermore, said polypeptide can be expressed using yeast, filamentous fungi, or the like, which are used for expression of recombinant proteins, as a host. Said polypeptide can also be obtained by a cell-free protein synthesis system.

For example, in the case of *E. coli,* a polynucleotide encoding a polypeptide consisting of DBL1X-ID2a or ID1-ID2a of VAR2CSA is synthesized with reference to the sequence registered in GenBank, and cloned into a vector that expresses protein using *E. coli* as a host. The codons of the introduced gene can also be mutated to codons preferentially used in the host organism, *E. coli.*

As an expression vector for expressing the VAR2CSA polypeptide, pET expression vector (Merck Millipore) having a T7 promoter, pGEX expression vector (Cytiva) or pLEAD expression vector (Nippon Gene) having a tac promoter, pCold expression vector (Takara Bio) having a promoter of the *E. coli* cold shock gene cspA, and the like can be enumerated, and pET expression vector is preferable. In the Examples below, pET-21b, a type of pET expression vector, was used. In the Examples below, a DNA fragment consisting of a polynucleotide encoding a polypeptide in which Twin-Strep-tag was linked to the N-terminus of the ID1-ID2a domain polypeptide and 3xFLAG-Tag was linked to the C-terminus was cloned into pET-21b. As a result, 6 X His-Tag is linked to the C-terminus of the final polypeptide sequence. The amino acid sequence of the VAR2CSA polypeptide derived from the FCR3 strain produced from the above expression vector is shown in SEQ ID NO: 8.

A host, *E. coli,* is transformed with a vector into which the DNA encoding the VAR2CSA polypeptide used in the present invention is cloned, and after culturing, by adding an appropriate inducer, the protein which is the cloned gene product can be expressed. This can be performed by well-known genetic engineering techniques and is also specifically described in the Examples below. To elaborate using the pET expression vector as an example, hosts include BL21(DE3), Tuner (tradename) (DE3), Rosetta (tradename) (DE3), Origami (tradename) (DE3), and SHuffle T7 Express, and IPTG is suitable as the inducer.

### 3. Immobilization of VAR2CSA polypeptide onto a support

As described later, the VAR2CSA polypeptide can be used by being immobilized on a support. Known methods can be used for immobilization of the VAR2CSA polypeptide onto a support. As the support, polymer beads, magnetic beads, wells of a microplate, or the like, which are widely used in the field of immunoassay, can be used. For example, by adding the VAR2CSA polypeptide diluted with an appropriate buffer to the wells of a polystyrene plate made for enzyme-linked immunosorbent assay (ELISA), the VAR2CSA polypeptide can be passively bound to the polystyrene surface.

For example, the VAR2CSA polypeptide can be immobilized onto the support surface by binding streptavidin to the support surface and causing binding between the streptavidin on the support surface and the Twin-Strep-tag arranged on the VAR2CSA polypeptide. As a plate-like support to which streptavidin is bound, IMMOBILIZER STREPTAVIDIN (Thermo Scientific Nunc, 436022), Streptavidin plate C8 transparent (Nunc, 236004), or Strep-Tactin (tradename) XT coated 96-well plate (IBS) can be used.

Furthermore, the VAR2CSA polypeptide can be immobilized onto the support surface by binding a nickel chelate to the support surface and causing binding between the nickel chelate on the support surface and the 6 X His-Tag peptide arranged on the VAR2CSA polypeptide. As a plate-like support to which a nickel chelate is bound, IMMOBILIZER NICKEL-CHELATE (Thermo Scientific Nunc, 436024) or Pierce (tradename) Nickel Coated Plates (Thermo Scientific) can be used.

More specifically, for example, the VAR2CSA polypeptide is diluted with PBS to 5 to 20 µg/mL, added to an ELISA plate, and incubated for 1 hour to 24 hours. After washing with a washing solution such as PBST, a blocking agent for ELISA plates is added and incubated at room temperature for 1 hour for blocking, whereby the VAR2CSA polypeptide can be immobilized. As the blocking agent, a commercially available blocking agent, for example, Blocking-one (Nacalai Tesque), Blocker (tradename) BLOTTO in TBS (Thermo Fisher Scientific), ELISA Coating Buffer 1X (abcam), or the like can be used. As a buffer for washing the support to which extracellular vesicles are bound, PBS containing 0.1% Tween 20 (PBST, Thermo Fisher Scientific) can be suitably used.

Magnetic beads can be used as the support for immobilizing the VAR2CSA polypeptide. The VAR2CSA polypeptide can be directly immobilized on magnetic beads, and suitable beads for them include Dynabeads (tradename) M-270 Epoxy (Thermo), Dynabeads M-280 Tosylactivated (Thermo), Dynabeads M-270 Carboxylic Acid (Thermo), and the like.

As another method, beads on the surface of which streptavidin is bound can be used. Examples of streptavidin-immobilized magnetic beads include Dynabeads M-270 Streptavidin (Thermo), Dynabeads (tradename) MyOne (tradename) Streptavidin C1 (Thermo), Dynal CELLection Biotin Binder (Thermo), Sera-Mag (tradename) SpeedBeads (tradename) Blocked Streptavidin particles (Sigma), MagStrep "type3" XT Beads (IBA), Magnosphere (tradename) MS300/Streptavidin (MBL), Strep-Tactin (tradename) AlphaLISA (tradename), Acceptor beads (Perkin Elmer), and the like. By mixing these magnetic beads with the VAR2CSA polypeptide provided with a Twin-Strep-tag peptide, the Twin-Strep-tag peptide arranged on the VAR2CSA polypeptide can be bound to the streptavidin on the bead surface.

As another method, beads on the surface of which nickel chelate or cobalt chelate is bound can be used. Examples of nickel chelate-immobilized magnetic beads include His60 Ni Magnetic Beads (Takara Bio), TALON (tradename), Magnetic Beads (Takara Bio), HisPur (tradename) Ni-NTA Magnetic Beads (Thermo), and the like. By mixing these magnetic beads and the VAR2CSA polypeptide, the 6 X His-Tag peptide arranged on the VAR2CSA polypeptide can be bound to the nickel or cobalt chelate on the bead surface.

Agarose or Sepharose can be used as the support for immobilizing the VAR2CSA polypeptide. Examples of streptavidin-immobilized support include Streptavidin-Agarose (Millipore), Streptavidin HP MultiTrap (Cytiva), Strep-Tactin (tradename) TACS Agarose (IBA), Strep-Tactin Sepharose (IBA), and the like.

Furthermore, agarose or Sepharose on which nickel chelate or cobalt chelate is immobilized can be similarly used. Examples of nickel chelate- or cobalt chelate-immobilized supports include Ni Sepharose (tradename) 6 Fast Flow (Cytiva), Ni-NTA Agarose (Thermo), TALON (tradename) Metal Affinity Resin (Takara Bio), and the like.

When immobilizing the VAR2CSA polypeptide onto a support, either the N-terminus or the C-terminus of the VAR2CSA polypeptide can be used for binding to the support. It is also possible to adjust the orientation of the VAR2CSA polypeptide immobilization onto the support by deciding whether to place/insert Tag peptides, such as the Twin-Strep-tag and 6xHis-Tag peptides, at the N-terminus or C-terminus of the VAR2CSA polypeptide, depending on the purpose and detection sensitivity. Since binding the VAR2CSA polypeptide to the support at the C-terminus rather than the N-terminus increases the amount of EVs captured by the VAR2CSA polypeptide, it is more preferable to use the C-terminus of the VAR2CSA polypeptide for immobilization onto the support.

### 4. Biological sample

The biological sample applied to the method of the present invention includes culture medium of cell lines and body fluids. The culture medium of a cell line is the supernatant from culturing cells. A cancer cell line is a cell line established by culturing cancer tissue, enabling subculture. A normal cell line is a cell line established by culturing tissue from a healthy person, enabling subculture; a primary culture line of tissue from a healthy person; or furthermore, a cell line immortalized by introducing immortalizing genes (hTERT, CDK4, CCND1, c-myc, etc.) into these cell lines, which are so-called non-cancer cell lines.

Examples of body fluids include blood (whole blood), plasma, serum, urine, stool, cerebrospinal fluid, pleural effusion, joint fluid, bone marrow, gastric fluid, feces, semen, prostatic fluid, saliva, sputum, pleural fluid, bile, pancreatic fluid, intestinal fluid, sweat, tears, nasal discharge, amniotic fluid, milk, aqueous humor, ascites, serous cavity fluid, lymph fluid, and the like. Preferable body fluids in the present invention include blood, plasma, serum, urine, saliva, and sputum, which are used as samples for liquid biopsy (body fluid biopsy).

Furthermore, the biological sample may be derived from an animal in a state of having developed cancer or in a tumor-bearing state, or derived from an animal serving as a control for comparison. It is preferably a specimen derived from a mammal, more preferably a specimen derived from a human, mouse, rat, rabbit, cow, pig, horse, dog, or cat, still more preferably a specimen derived from a human, dog, or cat, and most preferably a specimen derived from a human.

### 5. Method of capturing

The method of capturing cancer cell-derived extracellular vesicles according to the present invention includes a step of contacting a biological sample with a VAR2CSA polypeptide to bind the cancer cell-derived extracellular vesicles in the biological sample and the VAR2CSA polypeptide.

The binding step is conveniently and preferably performed by immobilizing the VAR2CSA polypeptide on a support as described above. The binding conditions for the cancer cell-derived extracellular vesicles and the VAR2CSA polypeptide are not particularly limited and can be set as appropriate, but, for example, it can be performed by incubating in a buffer at 4°C to room temperature for 1 hour to 18 hours.

The method of capturing of the present invention described above is applicable to use in therapy for suppressing cancer metastasis and for improving prognosis and QOL of cancer patients by removing cancer-derived extracellular vesicles from the blood of cancer patients using an extracorporeal circulation device.

It has been reported that cancer-derived extracellular vesicles are deeply involved in cancer metastasis and progression. Therefore, by removing cancer-derived extracellular vesicles from the blood of cancer patients using an extracorporeal circulation device, it is possible to stop cancer metastasis and cancer progression in cancer patients, and to improve patient's life extension and QOL.

In such an extracorporeal circulation device, an apparatus can be exemplified, which is an apparatus for removing cancer-derived extracellular vesicles from blood containing cells, extracellular vesicles, and plasma, wherein a hollow fiber through which blood passes has a plurality of pores, said pores are configured such that said extracellular vesicles of said blood can flow from the inside of said hollow fiber to the outside of said hollow fiber through said pores, and the VAR2CSA polypeptide immobilized on said hollow fiber is designed to comprise a functionalized surface that binds to said extracellular vesicles and removes them from said blood.

### 6. Method of detecting

The method of detecting cancer cell-derived extracellular vesicles according to the present invention includes a step of contacting a biological sample with a VAR2CSA polypeptide to bind the cancer cell-derived extracellular vesicles in the biological sample and the VAR2CSA polypeptide, and a step of detecting the cancer cell-derived extracellular vesicles bound to the VAR2CSA polypeptide. Note that quantification or semi-quantification can also be performed by the method of detecting of the present invention, but since quantification or semi-quantification inevitably involves detection, quantification and semi-quantification are also included within the scope of the method of detecting of the present invention.

Since this method of detecting is a method utilizing the specific binding between the VAR2CSA polypeptide and cancer cell-derived extracellular vesicles (that is, binding to cancer cell-derived extracellular vesicles, but not binding to normal cell-derived extracellular vesicles), as for the technique itself, techniques of well-known immunoassays can be applied. That is, techniques such as a sandwich method, an agglutination method, a competition method, an immunochromatography method, or the like can be applied. Among these, the sandwich method is preferable, and the sandwich method is also performed in the Examples below. To perform the sandwich method, another substance that binds to cancer cell-derived extracellular vesicles (a second binding substance) is necessary. As this second binding substance, an antibody or its antigen-binding fragment or an aptamer that binds to the surface of extracellular vesicles can be mentioned. It is known that proteins such as CD9, CD63, and CD81 are expressed on the surface of extracellular vesicles, and antibodies or their antigen-binding fragments or aptamers against these proteins can be used as the second binding substance that binds to extracellular vesicles. The antibody may be a monoclonal antibody or a polyclonal antibody as long as it is an antibody that can substantially detect extracellular vesicles expressing CD9, CD63, CD81, etc., and the animal species from which the antibody is obtained may be any of human, mouse, rabbit, goat, sheep, chicken, camel, llama, alpaca, shark, or ostrich.

Furthermore, as a second binding substance other than an antibody or its antigen-binding fragment, it is also possible to use the phosphatidylserine-binding domain of Tim1, Tim3, Tim4, or beta-2-glycoprotein 1 (β2GP1), which are proteins that bind to phosphatidylserine arranged on the outer side of the membrane of extracellular vesicles.

In the sandwich method, either one of the VAR2CSA polypeptide and the second binding substance is immobilized on a support, and the other is labeled, and the detection is performed by detecting said label bound to the support. Immobilization of the VAR2CSA polypeptide can be performed by the method described above. When immobilizing the second binding substance on a support, since the second binding substance is also a protein, it can be performed by the well-known methods described above.

For example, when the VAR2CSA polypeptide is immobilized on a support, the sandwich method can detect cancer cell-derived extracellular vesicles in a biological sample by, first, contacting the biological sample with the support on which the VAR2CSA polypeptide is immobilized, washing the support, then contacting with a labeled second binding substance, washing the support, and then detecting the label bound to the support via the cancer cell-derived extracellular vesicles.

Alternatively, the biological sample may first be contacted with the labeled second binding substance, then contacted with the support on which the VAR2CSA polypeptide is immobilized, and after washing, the label bound to the support via the cancer cell-derived extracellular vesicles may be detected (reverse method).

When the second binding substance is immobilized on a support, the sandwich method can detect cancer cell-derived extracellular vesicles in a biological sample by, first, contacting the biological sample with the support on which the second binding substance is immobilized, washing the support, then contacting with a labeled VAR2CSA polypeptide, washing the support, and then detecting the label bound to the support via the cancer cell-derived extracellular vesicles.

Alternatively, the biological sample may first be contacted with the labeled VAR2CSA polypeptide, then contacted with the support on which the second binding substance is immobilized, and after washing, the label bound to the support via the cancer cell-derived extracellular vesicles may be detected (reverse method), thereby detecting the cancer cell-derived extracellular vesicles in the biological sample.

Alternatively, the biological sample, the support on which the VAR2CSA polypeptide is immobilized, and the labeled second binding substance may be contacted simultaneously, and after washing, the label bound to the support via the cancer cell-derived extracellular vesicles may be detected.

Alternatively, the biological sample, the support on which the second binding substance is immobilized, and the labeled VAR2CSA polypeptide may be contacted simultaneously, and after washing, the label bound to the support via the cancer cell-derived extracellular vesicles may be detected.

As the label, well-known labels used in the field of immunoassay can be utilized. That is, well-known enzyme labels (e.g., HRP, ALP, etc.), fluorescent labels (e.g., FITC, PE, Alexa Fluor, etc.), and the like can be used. Since these labels are commercially available as kits, commercially available kits can be utilized. Furthermore, methods for binding a protein or aptamer to a label are also well-known and can be easily performed using commercially available kits.

Furthermore, a label for Amplified Luminescent Proximity Homogeneous Assay (Alpha) can also be adopted as the label. In this method, a label body (donor beads) that is excited by excitation light and a signal-generating label body (acceptor beads) that generates a signal by singlet oxygen generated by excitation are used, and the signal emitted from the signal-generating label body by the singlet oxygen generated from the excitation label body is detected.

In an embodiment using Alpha in the method of detecting of the present invention, either one of the VAR2CSA polypeptide and the second binding substance is immobilized on donor beads, and the other is bound to acceptor beads. When the donor beads and acceptor beads come into proximity via the binding of the VAR2CSA polypeptide and the cancer cell-derived extracellular vesicle, and the binding of the cancer cell-derived extracellular vesicle and the second binding substance, the aforementioned signal is generated, and this is detected.

The method of detecting using Alpha is included in the method of detecting of the present invention because the donor beads can be regarded as a support and the acceptor beads as a label. This method is also well-known, and kits therefor are also commercially available, so it can be easily performed using commercially available products.

In the method of capturing and the method of detecting of the present invention, the conditions for each "contact" are not particularly limited and can be set as appropriate, but, for example, it can be performed by incubating in a buffer at 4°C to room temperature for 1 hour to 18 hours. Furthermore, the detection of the label can be performed by a well-known method suitable for each label.

As a buffer for diluting the protein such as an antibody or an aptamer that detects the extracellular vesicles, for example, Blocking-one diluted by 20-fold with PBS containing 0.1% Tween 20, as well as commercially available antibody dilution buffers such as Can Get Signal (TOYOBO), ChonBlock Detection Antibody Dilution ELISA Buffer (Chondrex), Universal Antibody Dilution Buffer (tradename) (GeneTex), and the like can be used.

As a method of detecting the VAR2CSA polypeptide bound to the extracellular vesicles, it is also possible to utilize an antibody against VAR2CSA, and tag sequences arranged on the VAR2CSA polypeptide (Twin-Strep-tag peptide, 3 x FLAG-tag peptide, 6 x His-tag peptide, etc.. especially the 3 x FLAG-tag peptide and 6 x His-tag peptide bound to the C-terminus in the Examples of the present invention, etc.). When targeting the Twin-Strep-tag peptide, streptavidin that binds to the Twin-Strep-tag peptide can be utilized, and streptavidin labeled with HRP or the like is suitable. When targeting the 3 x FLAG-tag peptide, an anti-FLAG-tag antibody can be utilized, and an anti-FLAG-tag antibody labeled with HRP or the like is suitable. When targeting the 6 x His-tag peptide, an anti-His-Tag antibody can be utilized, and an anti-His-tag antibody labeled with HRP is suitable.

### 7. Cancer from which extracellular vesicles are derived

Examples of the cancer in the present invention include pancreatic cancer, biliary tract cancer, gallbladder cancer, bile duct cancer, lung cancer, colon cancer, prostate cancer, breast cancer, stomach cancer, cervical cancer, bladder cancer, testicular cancer, ovarian cancer, endometrial cancer, liver cancer, kidney cancer, head and neck cancer, esophageal cancer, pharyngeal cancer, osteosarcoma, sarcoma, glioma, lymphoma, leukemia, and the like, as long as the extracellular vesicles they express oncofetal chondroitin sulfate A on their EV surface layer. Glioma includes glioblastoma, and leukemia includes acute myeloid leukemia and acute lymphocytic leukemia.

### 8. Method of acquiring information on whether or not a subject has cancer

Furthermore, by the method of detecting of the present invention described above, it is also possible to quantify cancer cell-derived extracellular vesicles in a biological sample. When the quantified amount of VAR2CSA polypeptide-captured extracellular vesicles in a biological sample derived from a subject is significantly larger compared to the amount of VAR2CSA polypeptide-captured extracellular vesicles in a biological sample derived from a healthy person, it is also possible to determine that said subject has a high possibility of cancer. Furthermore, it is also possible to acquire data on the amount of VAR2CSA polypeptide-captured extracellular vesicles in biological samples of healthy persons in advance, set a threshold value in advance, and when the amount of VAR2CSA polypeptide-captured extracellular vesicles in a biological sample derived from a subject is greater than said threshold value, determine that said subject has a high possibility of cancer. It is also possible to make a similar determination using patients with benign diseases (including inflammatory diseases) that are not cancer as a comparison control instead of healthy persons.

The present invention also provides a kit used in the method of detecting of the present invention described above. The kit of the present invention is a kit comprising a VAR2CSA polypeptide and a second binding substance that binds to a surface of the extracellular vesicles, wherein: either one of (i) any one polypeptide of (A) to (C) described above and (ii) the second binding substance is immobilized on a support, and the other is labeled.

The kit of the present invention is preferably a kit for diagnosing whether or not a subject has cancer, comprising a VAR2CSA polypeptide and a second binding substance that binds to a surface of the extracellular vesicles, wherein: either one of (i) any one polypeptide of (A) to (C) described above and (ii) the second binding substance is immobilized on a support, and the other is labeled.

Alternatively, the kit of the present invention described above can also be used as a kit for capturing cancer cell-derived extracellular vesicles, and is a kit in which the VAR2CSA polypeptide is immobilized on a support. For example, by performing functional analysis of the captured cancer cell-derived extracellular vesicles, or by analyzing molecules contained in the extracellular vesicles, it can be used for studies to identify the cancer type, studies to elucidate the state of the cancer, and the like.

The kit of the present invention can include a buffer for diluting the biological sample before contacting it with the VAR2CSA polypeptide or the like, instructions for use, and so on.

### EXAMPLES

Hereinafter, the present invention will be described more specifically by way of Examples. However, these Examples are merely examples for specifically explaining the present invention and do not limit the technical scope of the present invention.

### Reference Example 1 Preparation of VAR2CSA polypeptide

A polynucleotide (SEQ ID NO: 7), optimized for *E. coli* codons, encoding a polypeptide (SEQ ID NO: 6) wherein Twin-Strep-tag (SEQ ID NO: 3) and a flexible linker (SEQ ID NO: 4) were arranged at the N-terminus of the amino acid sequence (SEQ ID NO: 1) of the ID1-ID2a domains of VAR2CSA of the *Plasmodium falciparum* FCR3 strain of GenBank accession number GU249598, and a 3xFLAG-tag (SEQ ID NO: 5) was arranged at the C-terminus of the same, was synthesized. This polynucleotide was inserted between NdeI and XhoI of the *E. coli* expression vector pET21b. Said vector was designated pET21b-VAR2-FCR3. The amino acid sequence of the polypeptide expressed from pET21b-VAR2-FCR3 is the one in which His-tag is added to the C-terminus of the polypeptide of SEQ ID NO: 6, and the sequence of that polypeptide is shown in SEQ ID NO: 8.

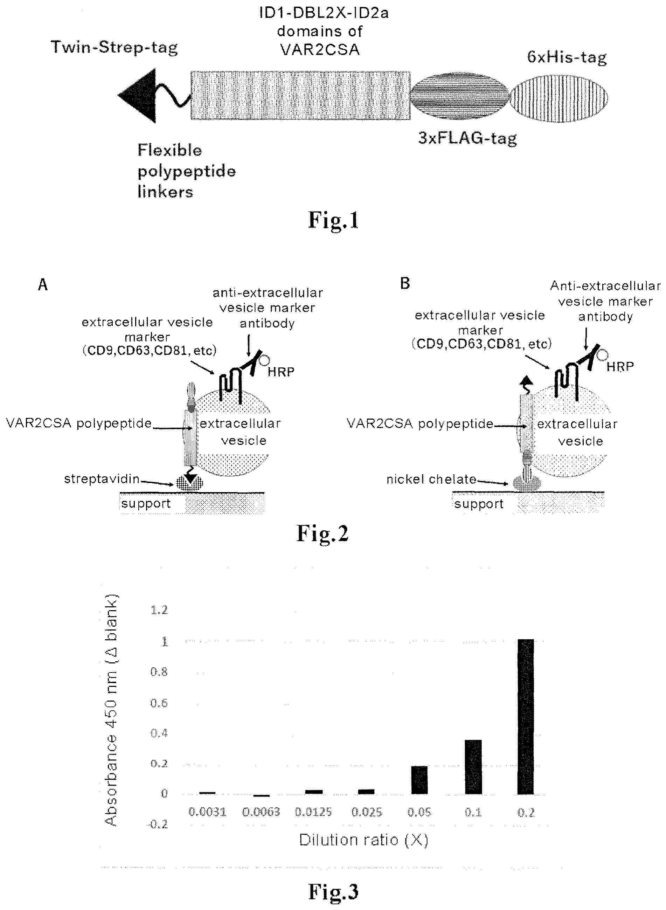

Furthermore, a schematic diagram of the structure of this VAR2CSA polypeptide is shown in FIG. 1.

The pET21b-VAR2-FCR3 plasmid DNA was introduced into the *E. coli* strain SHuffle T7 Express Competent E. *coli* (NEB; C3029J) to obtain transformants. A clone of this transformant was shake-cultured at 37°C for 18 hours in 10 mL of LB medium containing 100 µg/mL carbenicillin. 6 mL of the above culture solution was inoculated into 150 mL of LB medium (containing 100 µg/mL carbenicillin) and shake-cultured at 37°C until OD600 reached around 0.6. 1 mol/L IPTG was added to the culture solution to a final concentration of 0.1 mmol/L, and the mixture was shake-cultured at 20°C for about 20 hours. After completion of culture, centrifugation was performed at 6,000 x g for 15 minutes at 4°C to collect the *E. coli* pellet, which was then stored frozen at -80°C. The frozen *E. coli* pellet was thawed at room temperature, and 5 mL of B-PER (tradename) Bacterial Cell Lysis Reagent (Thermo Fisher Scientific; 89821) and 1/100 volume of protease inhibitor cocktail (Nacalai, 03969-21) were added per 1 g wet weight, followed by suspension by pipetting. After gentle stirring at room temperature for 15 minutes with a rotator, centrifugation was performed at 12,000 x g for 15 minutes at 4°C, and the supernatant was transferred to another new tube.

Purification was performed using a Strep-Tactin (tradename) XT 4Flow Starter Kit (IBA; 2-5998-000) according to the attached manual. The above extract was applied to a 1 mL Strep-Tactin (tradename) XT 4Flow column, washed with Buffer W attached to the kit, and eluted with Buffer BXT attached to the kit. The eluate was buffer-exchanged into PBS by a centrifugation method using PD MiniTrap G25 (cytiva; 28918007). The protein amount of the purified VAR2CSA was measured with a TaKaRa BCA Protein Assay Kit (Takara; T9300A), and the validity of the molecular weight was verified by SDS-PAGE. The biological activity of VAR2CSA was confirmed by its binding property to bovine decorin (Sigma; D8428) immobilized on an ELISA plate.

### Reference Example 2 Preparation of extracellular vesicles

The cell lines, media, and additives used in the present invention are shown in Table 2 for cancer cell lines and Table 3 for normal cell lines (non-cancer cell lines). The normal cells in the present invention are cells prepared by introducing immortalizing genes into primary cell lines obtained from ATCC or Lonza. Each cell line was cultured in a medium containing the additives listed in the tables until 70% to 80% confluency, the culture medium was removed, and the cells were cultured for 4 to 5 days in a medium for each cell containing 5% Exosome-Depleted FBS (ThermoFisher Scientific, A2720803).

The culture supernatant of each cell was collected, centrifuged at 300 x g for 5 minutes, and the supernatant was collected. This supernatant was centrifuged again at 8,000 x g for 30 minutes. The resulting supernatant was filtered through a 0.22 µm syringe filter (Millipore). Next, 40 mL of this filtered culture supernatant was concentrated to 1 mL using a centrifugal concentrator tube VIVASPIN 20 (Sartorius; VS2041) or Amicon Ultra-15 (Millipore; UFC910008) with a molecular weight cut-off of 100 kDa. Purification of extracellular vesicles in said concentrated sample was performed using MagCapture (tradename) Exosome Isolation Kit PS Ver. 2 (Fujifilm) according to the kit's attached manual. Briefly, 60 µL of Tim4-immobilized magnetic beads were added to 1 mL of the concentrated sample to adsorb the extracellular vesicles. After washing, the extracellular vesicles were eluted with 100 µL of elution buffer. To this eluate, 1/100 volume of extracellular vesicle blocking reagent, EV-Save (tradename) (Fujifilm), was added, and the mixture was aliquoted and stored at -80°C.

**[Table 2]**

| Organ | Cell line name | Species | Cancer name | Basal medium | Additive | Source |
|---|---|---|---|---|---|---|
| brain | U87-MG | human | glioblastoma | E-MEM | 10% FBS | ECACC 89081402 |
| pancreas | PANC-1 | human | pancreatic adenocarcinoma | D-MEM | 10% FBS | ATCC CRL-1469 |
| pancreas | KMP2 | human | Pancreatic cancer liver metastasis cell line | HamF12/RPMI1640 | 5% FBS | JCRB1427 |
| pancreas | MIA Paca-2 | human | pancreatic cancer | E-MEM | 10% FBS | JCRB0070 |
| prostate | LNCap | human | prostate cancer | RPMI-1640 | 10% FBS | RCB2144 |
| prostate | PC-3 | human | prostate cancer | RPMI-1640 | 10% FBS | JCRB9110 |
| lung | A549 | human | alveolar basal epithelium adenocarcinoma | E-MEM | 10% FBS | JCRB0076 |
| lung | NCI-H1703 | human | non-small cell lung cancer | RPMI-1640 | 10% FBS | ATCC CRL-5889 |
| lung | H69AR | human | small cell lung cancer | RPMI-1640 | 10% FBS | ATCC CRL-11351 |
| bone | MG63 | human | osteosarcoma | E-MEM | 10% FBS | JCRB/IFO50108 |
| esophagus | KYSE150 | human | esophageal cancer | HamF12/RPMI1640 | 5% FBS | JCRB1095 |
| esophagus | KYSE510 | human | esophageal cancer | HamF12/RPM11640 | 5% FBS | JCRB1436 |
| esophagus | KYSE1240 | human | esophageal cancer | HamF12/RPMI1640 | 5% FBS | JCRB1456 |
| stomach | HGC-27 | human | stomach cancer | E-MEM | 10% FBS | RCB0500 |
| stomach | AGS | human | stomach cancer | HamF 12 | 10% FBS | ECACC 89090402 |
| stomach | MKN45 | human | stomach cancer | RPMI-1640 | 10% FBS | JCRB0254 |
| colon | SW837 | human | colon cancer | Leibovitz's L-15 | 10% FBS | JCRB9115 |
| colon | SW480 | human | colon cancer | Leibovitz's L-15 | 10% FBS | ECACC 87092801 |
| colon | HTC-116 | human | colon cancer | D-MEM | 10% FBS | RCB2979 |
| colon | Caco-2 | human | colon cancer | E-MEM | 10% FBS | RCB0988 |
| blood | KG-1 | human | acute myeloid leukemia | RPMI-1640 | 10% FBS | JCRB0065 |

**[Table 3]**

| Organ | Cell line name | Cell type | Source | Immortalizing factor | Basal medium | Additive |
|---|---|---|---|---|---|---|
| lung | WI-38-h TERT | fetal lung fibroblast cell | ATCC | hTERT | D-MEM | 10% FBS |
| prostate | HPrEC-TKD | prostate epithelial cell | Lonza | hTERT+CDK4(R24C)+Cyclin D1 | RPMI1640 | 10%FBS+ 5 ng/mL EGF |
| prostate | HPrF-TKD | prostate fibroblast cell | Lonza | hTERT+CDK4(R24C)+Cyclin D1 | RPMI1640 | 10% FBS |
| bone | NHOst-TKD | osteoblast cell | Lonza | hTERT+CDK4(R24C)+Cyclin D1 | RPM11640 | 10% FBS |

### Reference Example 3 Preparation of antibodies

As antibodies for detecting extracellular vesicles, antibodies recognizing CD9, CD63, and CD81 expressed on the surface layer of extracellular vesicles were used. Specifically, anti-human CD9 rat monoclonal antibody (77B, Fujifilm), anti-human CD63 mouse monoclonal antibody (3-13, Fujifilm), and anti-human CD81 rat monoclonal antibody (9B, Fujifilm), which do not cross-react with bovine, were used.

100 µg of each of these antibodies was POD-labeled with Peroxidase Labeling Kit - NH2 (Dojindo). For detection of extracellular vesicles, said labeled antibodies were used at a 500-fold dilution.

### Reference Example 4 Detection of extracellular vesicles by ELISA

For detection of extracellular vesicles, an ELISA plate attached to the PS Capture (tradename) Exosome ELISA Kit (Fujifilm) or the CD9 / CD63 ELISA Kit (Cosmo Bio) was used, and the POD-labeled antibodies described in Reference Example 3 above were used as detection antibodies.

Briefly, the Tim4-immobilized plate of the PS Capture (tradename) Exosome ELISA Kit or the anti-CD9 antibody-immobilized plate of the CD9 / CD63 ELISA Kit was washed 3 times with the washing buffer attached to each kit, then the extracellular vesicle sample diluted with the reaction buffer attached to each kit was added to the wells of the plate, and after stirring for 30 seconds, incubated overnight at 4°C. After washing 5 times with the washing buffer attached to each kit, the POD-labeled antibody diluted by 500-fold with the reaction buffer attached to each kit was added, and after stirring for 30 seconds, incubated at room temperature. After 2 hours, the plate was washed 5 times with the washing buffer attached to each kit, the color-developing substrate solution of the ELISA POD Substrate TMB Kit HYPER (Nacalai) was added, and after stirring for 30 seconds, reacted for 5 to 10 minutes, and stop solution (1M sulfuric acid) was added, followed by stirring for 10 seconds to stop the reaction. Immediately after stopping the reaction, the absorbance (450 nm) was measured with a microplate reader. The absorbance is the value obtained by subtracting the blank value from each value.

### A. Method of capturing and detecting extracellular vesicles using VAR2CSA polypeptide in Examples

The VAR2CSA polypeptide prepared in Reference Example 1 was diluted with PBS to 10 µg/mL. 100 µL of the 10 µg/mL VAR2CSA polypeptide solution was added to each well of a streptavidin-coated plate (Nunc) and incubated overnight at 4°C. As another method, 100 µL of the 10 µg/mL VAR2CSA polypeptide solution was added to each well of Pierce (tradename) Nickel Coated Plates (Thermo Scientific) and incubated overnight at 4°C. After incubation, these plates were washed 3 times with PBST, and 200 µL of Blocking One (Nacalai) diluted by 5-fold with DW was added to each well and incubated for 1 hour for blocking.

The plate was washed 3 times with PBST, and extracellular vesicles diluted with Blocking One diluted by 20-fold with PBST were added, and after stirring for 30 seconds, incubated overnight at 4°C.

Detection of extracellular vesicles captured by the VAR2CSA polypeptide was performed as follows. After incubation, the plate was washed 5 times with PBST, the antibodies of Reference Example 3 diluted by 500-fold with antibody dilution buffer were added, and after stirring for 30 seconds, incubated for 2 hours. The plate was washed 5 times with PBST, the color-developing substrate solution of the ELISA POD Substrate TMB Kit HYPER (Nacalai) was added, and after stirring for 30 seconds, reacted for 5 to 10 minutes, and stop solution (1M sulfuric acid) was added, followed by stirring for 10 seconds to stop the reaction. Immediately after stopping the reaction, the absorbance (450 nm) was measured with a microplate reader. The absorbance is the value obtained by subtracting the blank value from each value. A schematic diagram illustrating the above analysis method is shown in FIG. 2.

### Example 1

### Detection of glioblastoma cell line-derived extracellular vesicles by VAR2CSA polypeptide

(1) Extracellular vesicles prepared by the method described in Reference Example 2 from the glioblastoma cell line U87-MG cells were serially diluted and captured and detected by the method described above in section A. Specifically, the purified EVs sample was serially diluted and added to each well of the VAR2CSA-immobilized plate. After incubation overnight at 4°C, the plate was washed 5 times with PBST, and an antibody solution of HRP-labeled anti-CD63 antibody diluted by 500-fold with dilution buffer was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 3.

### Comparative Example 1

### Detection of glioblastoma cell line-derived extracellular vesicles by anti-CD9 antibody

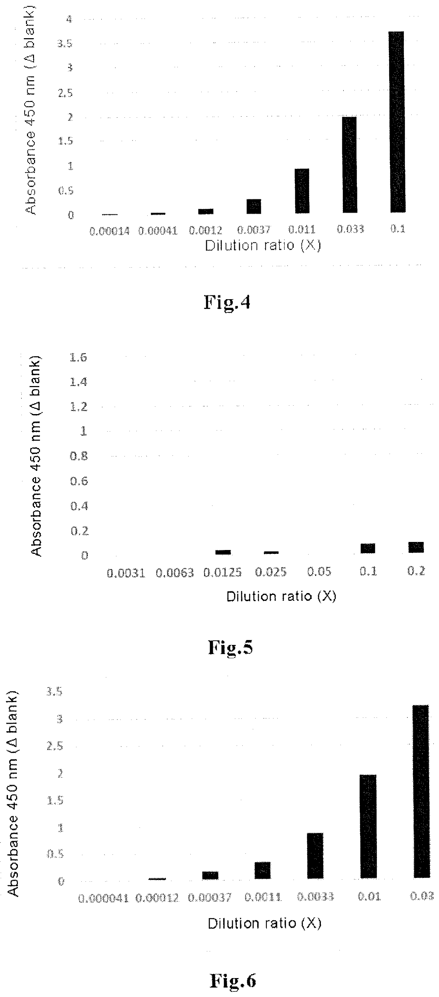

For comparison, the serially diluted U87-MG cell-derived purified extracellular vesicle sample was added to each well of an anti-CD9 antibody-immobilized plate. After incubation overnight at 4°C, the plate was washed 5 times with PBST, and an antibody solution of HRP-labeled anti-CD63 antibody diluted by 500-fold with dilution buffer was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 4.

The results of Example 1 and Comparative Example 1 above show that U87-MG cell-derived purified extracellular vesicles are captured by the VAR2CSA polypeptide and the anti-CD9 antibody and detected by the anti-CD63 antibody.

### Comparative Example 2

### Attempt of detection of normal fibroblast cell line-derived extracellular vesicles by VAR2CSA polypeptide

Extracellular vesicles prepared by the method described in Reference Example 2 from the fetal lung fibroblast cell line WI-38-hTERT were serially diluted and captured and detected by the method described above in section A.

Specifically, the purified EVs sample was serially diluted and added to each well of the VAR2CSA-immobilized plate. After incubation overnight at 4°C, the plate was washed 5 times with PBST, and an antibody solution of HRP-labeled anti-CD63 antibody diluted by 500-fold with dilution buffer was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 5.

### Comparative Example 3

### Detection of normal fibroblast cell line-derived extracellular vesicles by anti-CD9 antibody

For comparison, the serially diluted WI-38-hTERT cell-derived purified extracellular vesicle sample was added to each well of an anti-CD9 antibody-immobilized plate. After incubation overnight at 4°C, the plate was washed 5 times with PBST, and an antibody solution of HRP-labeled anti-CD63 antibody diluted by 500-fold with dilution buffer was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 6.

The results of Comparative Examples 2 and 3 above show that WI-38-hTERT cell-derived purified extracellular vesicles cannot be captured by the VAR2CSA polypeptide, but are captured by the anti-CD9 antibody and detected by the anti-CD63 antibody.

### Example 2

### Detection of pancreatic cancer cell line-derived extracellular vesicles by VAR2CSA polypeptide

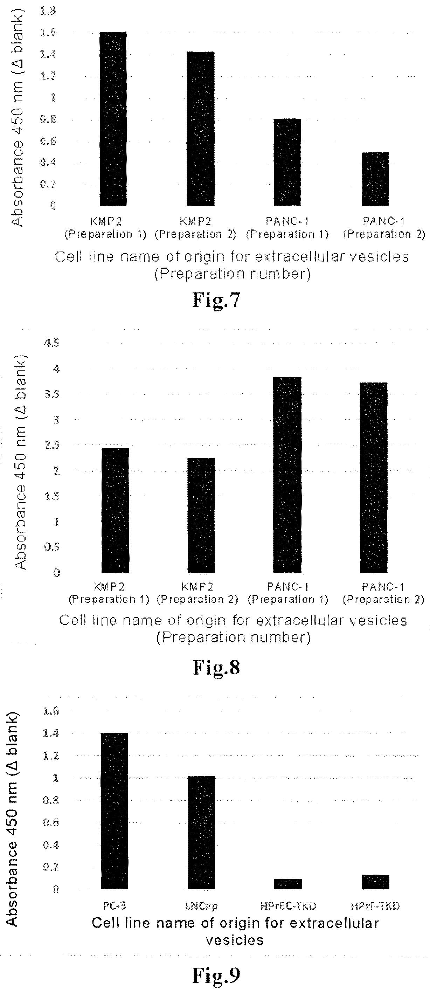

(1) Culture supernatants were collected from two separate cultures (Preparation 1 and Preparation 2) for each of the pancreatic cancer cell lines PANC-1 cells and KMP2 cells. Extracellular vesicles prepared by the method described in Reference Example 2 were diluted, and captured and detected by the method described above in section A. Specifically, the purified EVs sample was diluted 4-fold and added to each well of the VAR2CSA-immobilized plate. After incubation overnight at 4°C, the plate was washed 5 times with PBST, and an antibody cocktail solution, in which HRP-labeled anti-CD9 antibody and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 7.

### Comparative Example 4

### Detection of pancreatic cancer cell line-derived extracellular vesicles by Tim4 polypeptide

Furthermore, 40-fold diluted PANC-1 cell- and KMP2 cell-derived purified extracellular vesicle samples were added to each well of a Tim4-immobilized plate. After incubation overnight at 4°C, the plate was washed 5 times with washing solution, and an antibody cocktail solution, in which HRP-labeled anti-CD9 antibody and HRP-labeled anti-CD81 antibody were diluted by 500-fold respectively with dilution buffer, was added to each well and incubated at room temperature. After 2 hours, the plate was washed 5 times with washing solution, TMB substrate was added, and when color developed, stop solution was added, and the absorbance at 450 nm was immediately measured with a microplate reader. The results are shown in FIG. 8.

The results of Example 2 and Comparative Example 4 above show that PANC-1 cell- and KMP2 cell-derived purified extracellular vesicles are captured by the VAR2CSA polypeptide and Tim4, and detected by the cocktail antibody of anti-CD9 antibody and anti-CD81 antibody.

### Example 3

### Detection of prostate cancer cell line-derived extracellular vesicles by VAR2CSA polypeptide

Extracellular vesicles prepared by the method described in Reference Example 2 from the prostate cancer cell lines PC-3 cells and LNCap cells were diluted, and captured and detected by the method described above in section A. Specifically, the purified EVs sample was diluted by 4-fold and added to each well of the VAR2CSA-immobilized plate. After incubation overnight at 4°C, the plate was washed 5 times with PBST, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 9.

### Comparative Example 5

### Attempt of detection of normal prostate fibroblast cell line-derived extracellular vesicles by VAR2CSA polypeptide

An attempt was made to detect extracellular vesicles using normal cells, HPrEC-TKD cells and HPrF-TKD cells, instead of the prostate cancer cell lines in Example 3 above. The results are shown in FIG. 9.

### Comparative Example 6

Detection of prostate cancer cell line-derived extracellular vesicles by Tim4 polypeptide

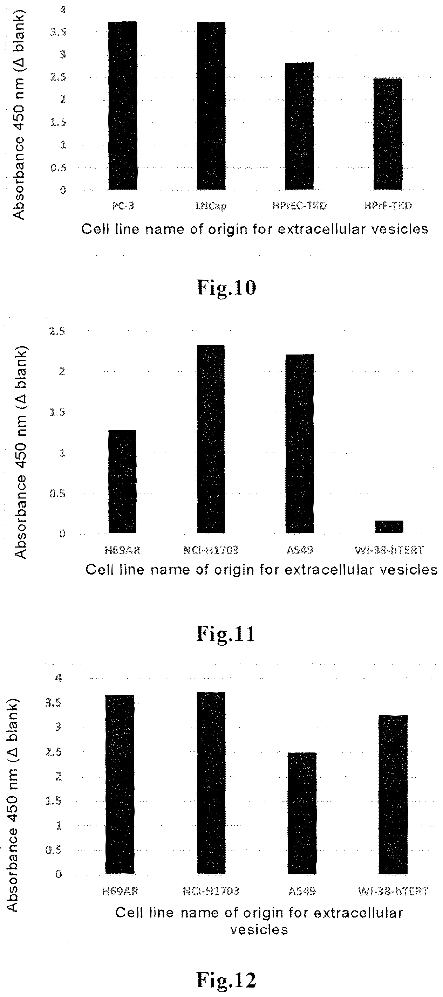

For comparison, 40-fold diluted purified extracellular vesicle samples derived from the prostate cancer cell lines PC-3 cells and LNCap cells were added to each well of a Tim4-immobilized plate. After incubation overnight at 4°C, the plate was washed 5 times with washing solution, and an antibody cocktail solution, in which IIRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with washing solution, TMB substrate was added, and when color developed, stop solution was added, and the absorbance at 450 nm was immediately measured with a microplate reader. The results are shown in FIG. 10.

### Comparative Example 7

### Detection of normal prostate fibroblast cell line-derived extracellular vesicles by Tim4 polypeptide

Extracellular vesicles were detected using normal cells, HPrEC-TKD cells and HPrF-TKD cells, instead of the prostate cancer cell lines in Comparative Example 6 above. The results are shown in FIG. 10.

The results of Example 3 and Comparative Examples 5 to 7 above show that PC-3 cell- and LNCap cell-derived purified extracellular vesicles are captured by the VAR2CSA polypeptide and Tim4 and detected by the cocktail antibody of anti-CD9 antibody, anti-CD63 antibody, and anti-CD81 antibody, whereas purified extracellular vesicles derived from normal cells HPrEC-TKD and HPrF-TKD are captured by Tim4 and detected by the cocktail antibody of anti-CD9 antibody, anti-CD63 antibody, and anti-CD81 antibody, but are not captured by the VAR2CSA polypeptide.

### Example 4

### Detection of lung cancer cell line-derived extracellular vesicles by VAR2CSA polypeptide

Extracellular vesicles prepared by the method described in Reference Example 2 from the lung cancer cell lines A549 cells, NCI-H1703 cells, and N69AR cells were diluted, and captured and detected by the method described above in section A. Specifically, the purified EVs sample was diluted 4-fold and added to each well of the VAR2CSA-immobilized plate. After incubation overnight at 4°C, the plate was washed 5 times with PBST, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 11.

### Comparative Example 8

### Attempt of detection of normal lung fibroblast cell line-derived extracellular vesicles by VAR2CSA polypeptide

An attempt was made to detect extracellular vesicles using normal cells, WI-38-hTERT cells, instead of the lung cancer cell lines in Example 4 above. The results are shown in FIG. 11.

### Comparative Example 9

### Detection of lung cancer cell line-derived extracellular vesicles by Tim4 polypeptide

For comparison, 40-fold diluted purified extracellular vesicle samples derived from A549 cells, NC1-H1703 cells, and N69AR cells were added to each well of a Tim4-immobilized plate. After incubation overnight at 4°C, the plate was washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with washing solution, TMB substrate was added, and when color developed, stop solution was added, and the absorbance at 450 nm was immediately measured with a microplate reader. The results are shown in FIG. 12.

### Comparative Example 10

### Detection of normal lung fibroblast cell line-derived extracellular vesicles by Tim4 polypeptide

Extracellular vesicles were detected using normal cells, WI-38-hTERT cells, instead of the lung cancer cell lines in Comparative Example 9 above. The results are shown in FIG. 12.

The results of Example 4 and Comparative Examples 8 to 10 above show that A549 cell-, NCI-H1703 cell-, and H69AR cell-derived purified extracellular vesicles are captured by the VAR2CSA polypeptide and Tim4 and detected by the cocktail antibody of anti-CD9 antibody, anti-CD63 antibody, and anti-CD81 antibody, whereas purified extracellular vesicles derived from normal cells WI-38-hTERT are captured by Tim4 and detected by the cocktail antibody of anti-CD9 antibody, anti-CD63 antibody, and anti-CD81 antibody, but are not captured by the VAR2CSA polypeptide.

### Example 5

### Detection of osteosarcoma cell line-derived extracellular vesicles by VAR2CSA polypeptide

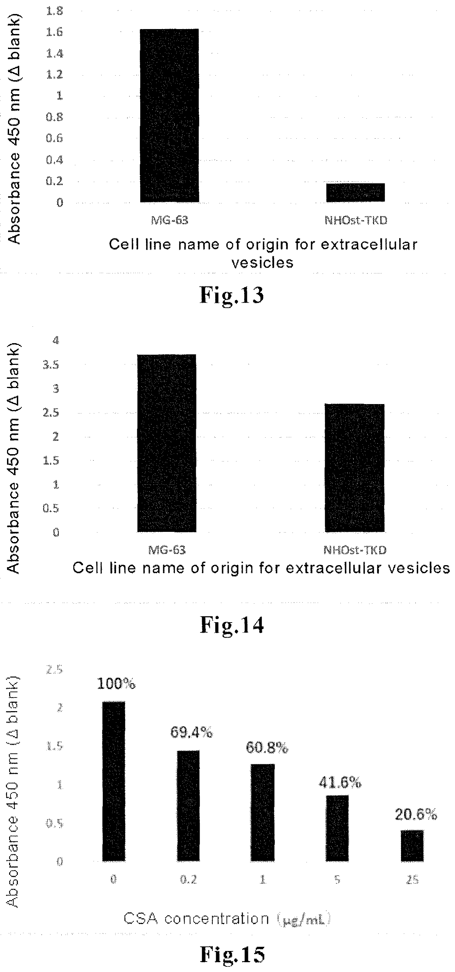

Extracellular vesicles prepared by the method described in Reference Example 2 from the osteosarcoma cell line MG-63 cells were diluted, and captured and detected by the method described above in section A. Specifically, the purified sample was diluted by 4-fold and added to each well of the VAR2CSA-immobilized plate. After incubation overnight at 4°C, the plate was washed 5 times with PBST, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 13.

### Comparative Example 11

### Attempt of detection of normal osteoblast cell line-derived extracellular vesicles by VAR2CSA polypeptide

An attempt was made to detect extracellular vesicles using normal osteoblasts, NHOst-TKD cells, instead of the osteosarcoma cell line in Example 5 above. The results are shown in FIG. 13.

### Comparative Example 12

### Detection of osteosarcoma cell line-derived extracellular vesicles by Tim4 polypeptide

For comparison, 40-fold diluted purified extracellular vesicle samples derived from the osteosarcoma cell line MG-63 cells were added to each well of a Tim4-immobilized plate. After incubation overnight at 4°C, the plate was washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with washing solution, TMB substrate was added, and when color developed, stop solution was added, and the absorbance at 450 nm was immediately measured with a microplate reader. The results are shown in FIG. 14.

### Comparative Example 13

### Detection of normal osteoblast cell line-derived extracellular vesicles by Tim4 polypeptide

Extracellular vesicles were detected using normal osteoblasts, NHOst-TKD cells, instead of the osteosarcoma cell line in Comparative Example 12 above. The results are shown in FIG. 14.

The results of Example 5 and Comparative Examples 11 to 13 above show that MG-63 cell-derived purified extracellular vesicles are captured by the VAR2CSA polypeptide and Tim4 and detected by the cocktail antibody of anti-CD9 antibody, anti-CD63 antibody, and anti-CD81 antibody, whereas purified extracellular vesicles derived from normal cells NHOst-TKD are captured by Tim4 and detected by the cocktail antibody of anti-CD9 antibody, anti-CD63 antibody, and anti-CD81 antibody, but are not captured by the VAR2CSA polypeptide.

### Example 6

### Detection of acute myeloid leukemia cell-derived cell line extracellular vesicles by VAR2CSA polypeptide and its inhibition by chondroitin sulfate A

Chondroitin sulfate (CSA) serially diluted was added to an extracellular vesicle sample prepared by the method described in Reference Example 2 from the acute myeloid leukemia cell-derived cell line KG-1 cells, and captured and detected by the method described above in section A. Specifically, the 4-fold diluted purified EVs sample was mixed so that the concentration of CSA became 0.2 µg/mL, 1 µg/mL, 5 µg/mL, and 25 µg/mL, and added to each well of the VAR2CSA-immobilized plate. After incubation overnight at 4°C, the plate was washed 5 times with PBST, and an antibody solution of HRP-labeled anti-CD63 antibody diluted by 500-fold with dilution buffer was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 15.

### Comparative Example 14

### Detection of acute myeloid leukemia cell-derived cell line extracellular vesicles by Tim4 polypeptide

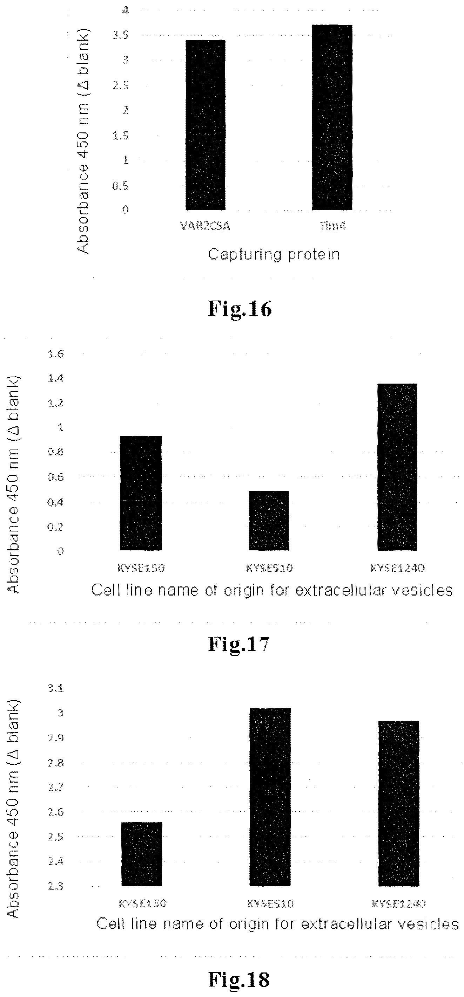

Furthermore, the extracellular vesicle sample prepared from KG-1 cells was diluted by 40-fold and added to the wells of a Tim4-immobilized plate, and after incubation overnight at 4°C, washed 5 times with washing solution, and HRP-labeled anti-CD63 antibody diluted 500-fold with dilution buffer was added to the wells and incubated at room temperature. After 2 hours, the plate was washed 5 times with washing solution, TMB substrate was added, and when color developed, stop solution was added, and the absorbance at 450 nm was immediately measured with a microplate reader. Evaluation using a VAR2CSA-immobilized plate was also performed in the same manner as in Example 6. The results are shown in FIG. 16.

The results of Example 6 and Comparative Example 14 above show that KG-1 cell-derived purified extracellular vesicles are captured by the VAR2CSA polypeptide and Tim4 and detected by the anti-CD63 antibody. Furthermore, since the capture of extracellular vesicles by the VAR2CSA polypeptide is inhibited by CSA, this suggests that the VAR2CSA polypeptide binds to CSA on the extracellular vesicles.

### Example 7

### Detection of esophageal cancer cell line-derived extracellular vesicles by VAR2CSA polypeptide

Extracellular vesicles prepared by the method described in Reference Example 2 from esophageal cancer cell lines with different differentiation types, KYSE150 cells (poorly differentiated), KYSE510 cells (well-differentiated), and KYSE1240 cells (moderately differentiated), were diluted, and captured and detected by the method described above in section A. Specifically, the purified EVs sample was diluted by 4-fold and added to each well of the VAR2CSA-immobilized plate. After incubation overnight at 4°C, the plate was washed 5 times with PBST, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 17.

### Comparative Example 15

### Detection of esophageal cancer cell line-derived extracellular vesicles by Tim4 polypeptide

Furthermore, 40-fold diluted purified extracellular vesicle samples derived from KYSE150 cells, KYSE510 cells, and KYSE1240 cells were added to each well of a Tim4-immobilized plate. After incubation overnight at 4°C, the plate was washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 18.

The results of Example 7 and Comparative Example 15 above show that KYSE150 cell-, KYSE510 cell-, and KYSE1240 cell-derived purified extracellular vesicles are captured by the VAR2CSA polypeptide and Tim4 and detected by the cocktail antibody of anti-CD9 antibody, anti-CD63 antibody, and anti-CD81 antibody. In esophageal cancer cells, cancer cells with a low degree of differentiation tend to have high malignancy, but even with esophageal cancer cell lines with different degrees of differentiation, their derived extracellular vesicles could be captured by the VAR2CSA polypeptide.

### Example 8

### Detection of stomach cancer cell line-derived extracellular vesicles by VAR2CSA polypeptide

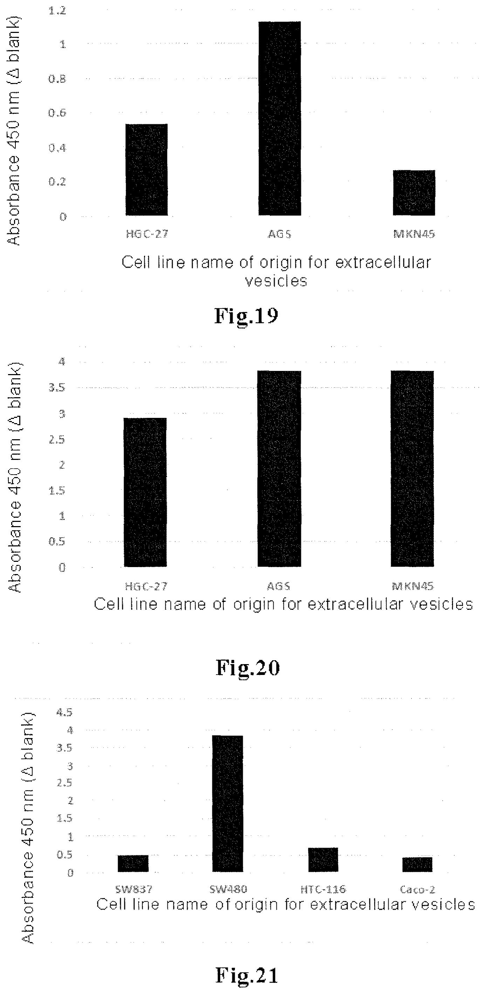

Extracellular vesicles prepared by the method described in Reference Example 2 from the stomach cancer cell lines HGC-27 cells, AGS cells, and MKN45 cells were diluted, and captured and detected by the method described above in section A. Specifically, the purified EV sample was diluted by 2-fold and added to each well of the VAR2CSA-immobilized plate. After incubation overnight at 4°C, the plate was washed 5 times with PBST, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 19.

### Comparative Example 16

### Detection of stomach cancer cell line-derived extracellular vesicles by Tim4 polypeptide

Furthermore, 20-fold diluted purified extracellular vesicle samples derived from HGC-27 cells, AGS cells, and MKN45 cells were added to each well of a Tim4-immobilized plate. After incubation overnight at 4°C, the plate was washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 20.

The results of Example 8 and Comparative Example 16 above show that HGC-27 cell-, AGS cell-, and MKN45 cell-derived purified extracellular vesicles are captured by the VAR2CSA polypeptide and Tim4 and detected by the cocktail antibody of anti-CD9 antibody, anti-CD63 antibody, and anti-CD81 antibody.

### Example 9

### Detection of colon cancer cell line-derived extracellular vesicles by VAR2CSA polypeptide

Extracellular vesicles prepared by the method described in Reference Example 2 from the colon cancer cell lines SW837 cells, SW480 cells, HTC-116 cells, and Caco-2 cells were diluted, and captured and detected by the method described above in section A. Specifically, the purified EV sample was diluted by 2.5-fold and added to each well of the VAR2CSA-immobilized plate. After incubation overnight at 4°C, the plate was washed 5 times with PBST, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 21.

### Comparative Example 17

### Detection of colon cancer cell line-derived extracellular vesicles by Tim4 polypeptide

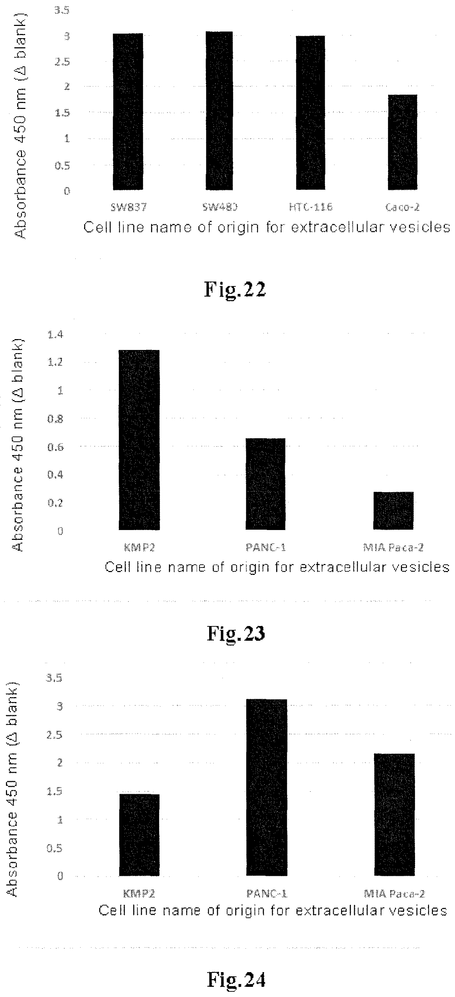

Furthermore, 25-fold diluted purified extracellular vesicle samples derived from SW837 cells, SW480 cells, HTC-116 cells, and Caco-2 cells were added to each well of a Tim4-immobilized plate. After incubation overnight at 4°C, the plate was washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 22.

The results of Example 9 and Comparative Example 17 above show that SW837 cell-, SW480 cell-, HTC-1 16 cell-, and Caco-2 cell-derived purified extracellular vesicles are captured by the VAR2CSA polypeptide and Tim4 and detected by the cocktail antibody of anti-CD9 antibody, anti-CD63 antibody, and anti-CD81 antibody. In particular, it was suggested that the target of the VAR2CSA polypeptide is highly expressed on extracellular vesicles derived from SW480 cells. Furthermore, the colon cancer's extracellular vesicle marker CD147 is not expressed in Caco-2, which has low malignancy, and cannot be detected by an assay system using an anti-CD9 antibody and an anti-CD147 antibody (WO2014/167969), but it can be detected by the system capturing with the VAR2 polypeptide.

### Example 10

### Detection of EpCAM-negative pancreatic cancer cell-derived extracellular vesicles by VAR2CSA polypeptide

Epithelial cancers are often EpCAM-positive, and EpCAM is frequently used as a marker for cancer-derived extracellular vesicles. However, many cancers are EpCAM-negative, and it is a problem that they cannot be detected with an anti-EpCAM antibody in the EpCAM-negative case.

Therefore, we investigated whether the VAR2CSA polypeptide can capture extracellular vesicles derived from EpCAM-negative cell lines. Extracellular vesicle samples prepared from the culture supernatants of EpCAM-positive KMP2 cells and PANC-1 cells, and EpCAM-negative MIA Paca-2 cells were diluted by 2.5-fold and added to each well of the VAR2CSA polypeptide-immobilized plate. After incubation overnight at 4°C, the plate was washed 5 times with PBST, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 23.

### Comparative Example 18

### Detection of EpCAM-negative pancreatic cancer cell-derived extracellular vesicles by Tim4 polypeptide

Furthermore, 25-fold diluted purified extracellular vesicle samples derived from KMP2 cells, PANC-1 cells, and MIA Paca-2 cells were added to each well of a Tim4-immobilized plate. After incubation overnight at 4°C, the plate was washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 24.

The results of Example 10 and Comparative Example 18 above show that EpCAM-positive KMP2 cell- and PANC-1 cell-, and EpCAM-negative MIA Paca-2 cell-derived purified extracellular vesicles are captured by the VAR2CSA polypeptide and Tim4 and detected by the cocktail antibody of anti-CD9 antibody, anti-CD63 antibody, and anti-CD81 antibody.

### Example 11

### Detection with VAR2CSA polypeptide of glioblastoma cell line-derived extracellular vesicles captured by anti-CD63 antibody

Glioblastoma cell line U87-MG cell culture supernatant was serially diluted by 2-fold with sample dilution buffer, and each diluted sample was added to an anti-CD63 antibody-immobilized plate (no cross-reactivity to bovine) (CD63-Capture Human Exosome ELISA Kit; manufactured by Fujifilm).

After incubation for 18 hours at 4°C and washing with washing buffer, 5 µg/mL of VAR2CSA polypeptide solution was added and incubated for 2 hours at room temperature.

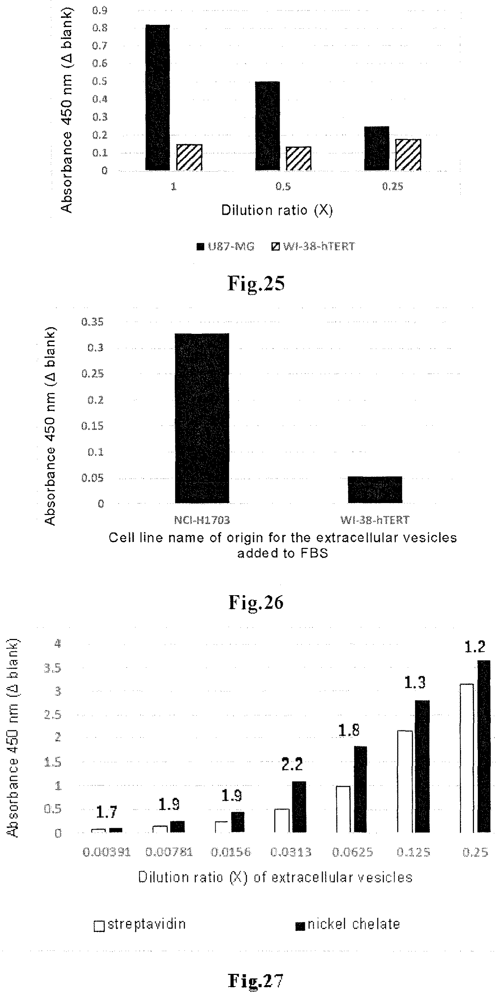

After washing, HRP-anti-FLAG antibody (manufactured by Sigma-Aldrich), which recognizes the 3xFLAG-tag peptide added to the VAR2CSA polypeptide, was added and further incubated for 2 hours at room temperature. After washing, TMB solution, which is a peroxidase color-developing substrate, was added and reacted for 10 minutes. Color development stop solution was added thereto to stop the reaction, and the absorbance at 450 nm of each well was immediately measured. The absorbance in the figure is represented as the value obtained by subtracting the blank value with culture medium from each value. The results are shown in FIG. 25.

### Comparative Example 19

### Attempt of detection with VAR2CSA polypeptide of normal fetal lung fibroblast cell line-derived extracellular vesicles captured by anti-CD63 antibody

An attempt was made to detect extracellular vesicles using normal fetal lung fibroblasts, WI-38-hTERT cells, instead of the glioblastoma cell line in Example 11 above. The results are shown in FIG. 25.

The results of Example 11 and Comparative Example 19 above show that, for extracellular vesicles captured by an antibody against an extracellular vesicle marker, extracellular vesicles derived from cancer cells can be detected with the VAR2CSA polypeptide, but extracellular vesicles derived from normal cells cannot be detected with the VAR2CSA polypeptide (only background-level signals detected at any extracellular vesicle amounts).

### Example 12

### Detection of lung cancer cell line-derived extracellular vesicles added to serum

For lung cancer cell line NCI-H1703 cell-derived purified extracellular vesicles and normal fetal lung fibroblast cell line WI-38-hTERT cell-derived purified extracellular vesicles, 25 µL of each was added to and mixed with 75 µL of exosome-depleted FBS. The sample was added to a plate on which VAR2CSA polypeptide was immobilized.

After incubation for 18 hours at 4°C and washing with washing buffer, a mixed antibody solution of HRP-labeled anti-CD9 antibody, HRP-labeled anti-CD63 antibody, and HRP-labeled anti-CD81 antibody, diluted with antibody dilution buffer, was added and incubated for 2 hours at room temperature.

After washing, TMB solution, which is a peroxidase color-developing substrate, was added and reacted for 10 minutes. Color development stop solution was added thereto to stop the reaction, and the absorbance at 450 nm of each well was immediately measured. The absorbance is represented as the value obtained by subtracting the blank value from each value. The results are shown in FIG. 26.

The results of Example 12 above show that cancer cell-derived extracellular vesicles added to serum can be detected.

Furthermore, the above results are summarized in Table 4. In the table, O indicates that it was captured, and x indicates that it was not captured. It is shown that cancer cell-derived extracellular vesicles are captured by VAR2CSA, but normal cell-derived extracellular vesicles are not captured.

**[Table 4]**

| Site | Cancer type/normal cell | Cell name | Capturing molecule VAR2 | Capturing molecule Tim-4/CD63/CD9 |
|---|---|---|---|---|
| brain | glioblastoma | U87-MG | O | O |
| pancreas | pancreatic cancer | KMP2 | O | O |
| | | PANC-1 | O | O |
| | | MIA Paca-2 | O | O |
| prostate | prostate cancer | PC-3 | O | O |
| | | LNCap | O | O |
| | prostate epithelial cell | HPrEC | × | O |
| | prostate fibroblast cell | HPrF | x | O |
| lung | lung cancer | H1703 | O | O |
| | | H69AR | O | O |
| | | A549 | O | O |
| | fetal fibroblast cell | WI-38 | × | O |
| bone | osteosarcoma | MG-63 | O | O |
| | osteoblast cell | NHOst | × | O |
| esophagus | esophageal cancer | KYSE150 | O | O |
| | | KYSE510 | O | O |
| | | KYSE1240 | O | O |
| stomach | stomach cancer | HGC-27 | O | O |
| | | AGS | O | O |
| | | MKN45 | O | O |
| colon | colon cancer | SW837 | O | O |
| | | SW480 | O | O |
| | | HTC-116 | O | O |
| | | Caco-2 | O | O |
| blood | leukemia | KG-1 | O | O |

### Example 13

### Comparison of extracellular vesicle capture rate due to differences in the method of immobilizing VAR2CSA polypeptide onto support

The extracellular vesicle capture efficiency of the VAR2CSA polypeptide was compared between a method of immobilizing the N-terminal side of the VAR2CSA polypeptide to the plate (FIG. 2A) and a method of immobilizing the C-terminal side of the VAR2CSA polypeptide to the plate (FIG. 2B). To capture the N-terminal side of the VAR2CSA polypeptide, the VAR2CSA polypeptide was added to a streptavidin-coated plate. To capture the C-terminal side of the VAR2CSA polypeptide, the VAR2CSA polypeptide was added to a nickel chelate-coated plate (see section A).

Purified extracellular vesicles from the lung cancer cell line NCI-H1703 cells were serially diluted and added to each plate. After incubation for 18 hours at 4°C and washing with washing buffer, a mixed antibody solution of HRP-anti-CD9 antibody, HRP-anti-CD63 antibody, and HRP-anti-CD81 antibody, diluted with antibody dilution buffer, was added and incubated for 2 hours at room temperature. After washing, TMB solution, which is a peroxidase color-developing substrate, was added and reacted for 10 minutes. Color development stop solution was added thereto to stop the reaction, and the absorbance at 450 nm of each well was immediately measured. The absorbance in the figure is the value obtained by subtracting the blank value from each value. The results are shown in FIG. 27. The numerical value in the figure is the ratio of the value with the nickel chelate-coated plate to the value with the streptavidin-coated plate. It was demonstrated that immobilizing the C-terminal side of VAR2CSA onto a nickel chelate-coated plate increased extracellular vesicle capture efficiency by between 1.2-fold to 2.2-times.

### Reference Example 4

### Preparation of VAR2CSA polypeptides from strains other than FCR3 strain

Polynucleotides were synthesized, optimized for *E. coli* codons, encoding polypeptides in which Twin-Strep-tag (SEQ ID NO: 3) and a flexible linker (SEQ ID NO: 4) were arranged at the N-terminus, and 3xFLAG-tag (SEQ ID NO: 5) and 6xHis-tag (HHHHHH) were arranged at the C-terminus, of the amino acid sequences (SEQ ID NO: 9, SEQ ID NO: 12, SEQ ID NO: 13, and SEQ ID NO: 14, respectively) of the ID1-ID2a domain of VAR2CSA of *Plasmodium falciparum* FCR3 strains from GenBank accession numbers EUR69480 (7G8), OK346630 (M0920), JQ247428 (3D7), and OK346631 (M.Camp; sometimes abbreviated as MC). These polynucleotides were inserted between NdeI and NotI of the *E. coli* expression vector pET21b. Said vectors were designated pET21b-VAR2-7G8, pET21b-VAR2-M0920, pET21b-VAR2-3D7, and pET21b-VAR2-M.Camp, respectively. The amino acid sequences of the polypeptides expressed from these vectors are shown in SEQ ID NO: 64, 65, 66, and 67. Furthermore, Table 5 shows the calculated molecular weights of these polypeptides.

**[Table 5]**

| *Plasmodium falciparum* strain name from which VAR2CSA was derived | SEQ ID NO: | calculated molecular weight |
|---|---|---|
| 7G8 | 64 | 79004.26 |
| FCR3 | 8 | 79839.93 |
| 3D7 | 66 | 79565.26 |
| M.Camp (MC) | 67 | 81875.21 |
| M0920 | 65 | 79565.86 |

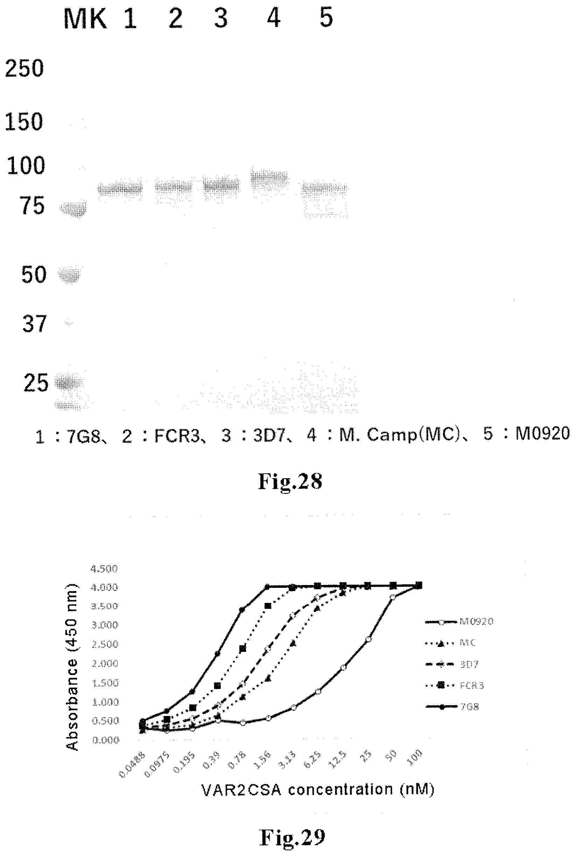

Expression of the genes encoded by these vectors and purification of the expressed proteins were performed according to Reference Example 1. The purified VAR2CSA polypeptides of the five strains were analyzed by SDS polyacrylamide gel electrophoresis (SDS-PAGE) at 2 µg/lane. The results are shown in FIG. 28. This result shows that the five types of VAR2CSA polypeptides have high purity.

### Reference Example 5

### Binding analysis of 5 types of VAR2CSA polypeptides

Bovine decorin (Sigma; D8428) was immobilized on an ELISA plate at a concentration of 2 µg/mL and at 100 µg/mL. The five types of VAR2CSA polypeptides were serially diluted by 2-fold from a concentration of 100 nM. This diluted sample was added to the bovine decorin-immobilized ELISA plate, and after washing, detected with an antibody against the 3xFLAG-tag in the VAR2CSA polypeptide (HRP-labeled anti-FLAG M2 antibody, Sigma-Aldrich; A8592). The results are shown in FIG. 29. These results showed that the binding affinity to bovine decorin is strongest in the following order: 7G8 > FCR3 > 3D7 > M. Camp (MC) > M0920.

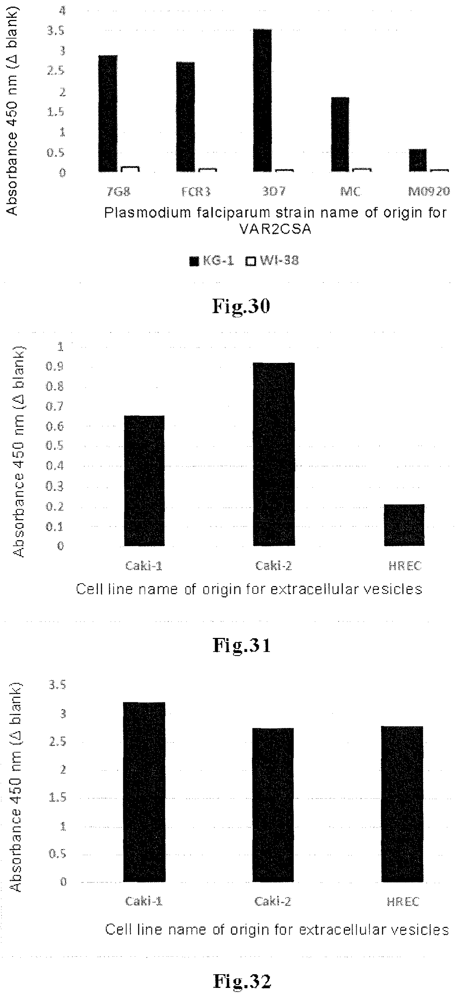

Next, the binding properties between the various *Plasmodium falciparum* strain-derived VAR2CSA polypeptides and EVs were investigated. Various VAR2CSA polypeptides at a concentration of 100 nM were added to a streptavidin plate (Nunc; 236004) to immobilize the VAR2CSA polypeptides. To the wells of this plate, 100 µL of 4-fold diluted purified EVs derived from the leukemia-derived cell line KG-1 cells and the normal fetal lung fibroblast cell line WI-38-hTERT cells were added, and after overnight incubation at 4°C, washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The absorbance in the figure is the value obtained by subtracting the blank value from each value. The results are shown in FIG. 30.

VAR2CSA polypeptides derived from all strains captured EVs derived from cancer cell lines but failed to capture EVs derived from normal cell lines. In addition, the VAR2CSA polypeptide derived from the M0920 strain showed a lowest binding affinity to the cancer-derived EVs. This result reflected the binding properties to decorin.

### Reference Example 6

### Preparation of extracellular vesicles

The cell lines, media, and additives additionally used in the present invention are shown in Table 6 for cancer cell lines and Table 7 for normal cell lines (non-cancer cell lines). Extracellular vesicles were prepared from the culture supernatant by the same method as in Reference Example 2.

**[Table 6]**

| Organ | Cell line name | Species | Cancer name | Basal medium | Additive | Source |
|---|---|---|---|---|---|---|
| breast | MDA-MB-453 | human | breast cancer | Leibovitz's L-15 Medium | 10% FBS | RCB1192 |
| breast | MDA-MB-231 | human | breast cancer | Leibovitz's L-15 Medium | 10% FBS | ATCC HTB-26 |
| breast | T47D | human | breast cancer | RPMI-1640 | 10% FBS | ATCC HTB-133 |
| kidney | Caki-1 | human | kidney cancer | E-MEM | 10% FBS | JCRB0801 |
| kidney | Caki-2 | human | kidney cancer | McCoy's 5a | 10% FBS | EC93120819 |
| ovary | OVCAR 3 | human | ovary cancer | RPMI-1640 | 10% FBS | RCB2135 |
| bladder | T24 | human | bladder cancer | E-MEM | 10% FBS | JCRB0711 |
| liver | HepG2 | human | liver cancer | D-MEM | 10% FBS | JCRB1054 |
| gallbladder | TYGBK-8 | human | gallbladder cancer | RPM11640/ HamF12 | 10% FBS | JCRB1590 |
| bile duct | HuCCT1 | human | bile duct cancer | RPMI-1640 | 10% FBS | JCRB0425 |
| bile duct | TFK-1 | human | bile duct cancer | RPMI-1640 | 10% FBS | RCB2537 |
| blood | NALM-6 | human | acute lymphocytic leukemia | RPMI-1640 | 10% FBS | RCB1933 |
| blood | KG-1 | human | acute myeloid leukemia | RPMI-1640 | 10% FBS | JCRB0065 |

**[Table 7]**

| Organ | Cell line name | Cell type | Source | Immortalizing factor | Basal medium | Additive |
|---|---|---|---|---|---|---|
| kidney | HREC-TKD | kidney epithelial cell | Lonza | hTERT+CDK4(R24C)+Cyclin D1 | D-MEM | 10% FBS |
| breast | HMEC-TKD | breast epithelial cell | Lonza | hTERT+CDK4(R24C)+Cyclin D1 | MEBM^{™} Mammary Epithelial Cell Basal Medium | MEGM^{™} SingleQuots^{™} |
| bronchi | HBSMC-TKD | bronchial smooth muscle cell | Lonza | hTERT+CDK4(R24C)+Cyclin D1 | RPMI16401HamF12 | 10% FBS |
| aorta | HAEC-TKD | aortic endothelial cell | Lonza | HTERT+CDK4(R24C)+Cyclin D1 | RPMI1640/HamF12 | 10%FBS+5 ng/mL VEGF |

Using these purified EVs samples, the binding properties of the VAR2CSA polypeptide and EVs derived from various cell lines were analyzed.

### Example 14

### Detection of extracellular vesicles derived from kidney cancer cell lines by VAR2CSA polypeptide

Extracellular vesicles prepared from the kidney cancer cell lines Caki-1 cells and Caki-2 cells by the method of Reference Example 6 were diluted, and captured and detected by the method described above in section A. Specifically, the purified EVs sample was diluted by 2.5-fold and added to each well of a VAR2CSA-immobilized plate. The VAR2CSA-immobilized plate was prepared by adding the VAR2CSA polypeptide to a nickel chelate-coated plate. After incubation overnight at 4°C, and washing 5 times with PBST, an antibody cocktail solution, in which an HRP-labeled anti-CD9 antibody, an HRP-labeled anti-CD63 antibody, and an HRP-labeled anti-CD81 antibody were diluted by 500-fold respectively with a dilution buffer, was added to each well and incubated at room temperature. After 2 hours, the plate was washed 5 times with PBST, a TMB substrate was added, and when color developed, a reaction stop solution was added, and the absorbance at 450 nm was immediately measured with a microplate reader. The absorbance in the figure is the value obtained by subtracting the blank value from each value. The results are shown in FIG. 31.

### Comparative Example 20

### Attempt of detection of extracellular vesicles derived from HREC-TKD cells, which are a normal kidney epithelial strain, by VAR2CSA polypeptide

Detection of extracellular vesicles was attempted using HREC-TKD cells, which are a normal kidney epithelial strain, instead of the kidney cancer cell lines in Example 14 above. The results are shown in FIG. 31.

### Comparative Example 21

### Detection of extracellular vesicles derived from kidney cancer cell lines by Tim4 polypeptide

In addition, Caki-1 cell and Caki-2 cell purified extracellular vesicle diluted by 25-fold were added to each well of a Tim4-immobilized plate. After incubation overnight at 4°C, and washing 5 times with a washing solution, an antibody cocktail solution, in which an HRP-labeled anti-CD9 antibody, an HRP-labeled anti-CD63 antibody, and an HRP-labeled anti-CD81 antibody were diluted by 500-fold respectively with a dilution buffer, was added to each well and incubated at room temperature. After 2 hours, the plate was washed 5 times with a washing solution, a TMB substrate was added, and when color developed, a reaction stop solution was added, and the absorbance at 450 nm was immediately measured with a microplate reader. The absorbance in the figure is the value obtained by subtracting the blank value from each value. The results are shown in FIG. 32.

### Comparative Example 22

### Detection of extracellular vesicles derived from HREC-TKD cells, which are a normal kidney epithelial strain, by Tim4 polypeptide

Extracellular vesicles were detected using HREC-TKD cells, which are a normal kidney epithelial strain, instead of the kidney cancer cell lines in Comparative Example 21 above. The results are shown in FIG. 32.

The results of Example 14 and Comparative Examples 20 to 22 above show that purified extracellular vesicles derived from Caki-1 cells and Caki-2 cells are captured by the VAR2CSA polypeptide and Tim4 and detected by the cocktail antibody of the anti-CD9 antibody, anti-CD63 antibody, and anti-CD81 antibody, whereas purified extracellular vesicles derived from HREC-TKD are captured by Tim4 and detected by the cocktail antibody of the anti-CD9 antibody, anti-CD63 antibody, and anti-CD81 antibody, but are not captured by the VAR2CSA polypeptide.

### Example 15

### Detection of extracellular vesicles derived from breast cancer cell lines by VAR2CSA polypeptide

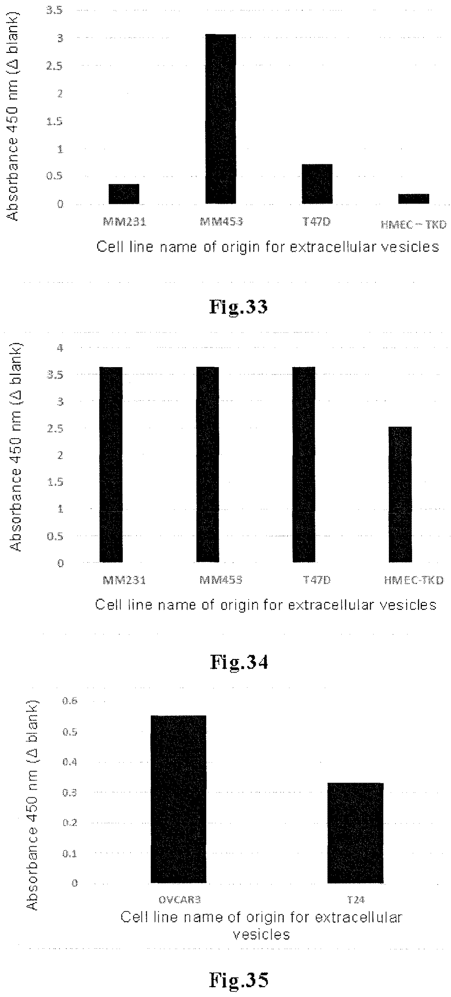

Extracellular vesicles prepared by the method of Reference Example 6 from breast cancer cell lines MDA-MB-231 cells (also called as MM231 cells), MDA-MB-453 cells (also called as MM453 cells), and T47D cells were diluted, and captured and detected by the method described above in section A. Specifically, the purified EVs sample was diluted by 2.5-fold and added to each well of a VAR2CSA-immobilized plate. The VAR2CSA-immobilized plate was prepared by adding the VAR2CSA polypeptide to a nickel chelate-coated plate. After incubation overnight at 4°C, and washing 5 times with PBST, an antibody cocktail solution, in which an HRP-labeled anti-CD9 antibody, an HRP-labeled anti-CD63 antibody, and an HRP-labeled anti-CD81 antibody were diluted by 500-fold respectively with a dilution buffer, was added to each well and incubated at room temperature. After 2 hours, the plate was washed 5 times with PBST, a TMB substrate was added, and when color developed, a reaction stop solution was added, and the absorbance at 450 nm was immediately measured with a microplate reader. The absorbance in the figure is the value obtained by subtracting the blank value from each value. The results are shown in FIG. 33.

### Comparative Example 23

Attempt of detection of extracellular vesicles derived from a normal mammary epithelial cell line by VAR2CSA polypeptide

Detection of extracellular vesicles was attempted using HMEC-TKD cells, which are a normal mammary epithelial cell line, instead of the breast cancer cell lines in Example 15 above. The results are shown in FIG. 33.

### Comparative Example 24

### Detection of extracellular vesicles derived from breast cancer cell lines by Tim4 polypeptide

In addition, purified extracellular vesicle samples derived from MDA-MB-231 cells, MDA-MB-453 cells, and T47D cells diluted by 25-fold were added to each well of a Tim4-immobilized plate. After incubation overnight at 4°C, and washing 5 times with a washing solution, an antibody cocktail solution, in which an HRP-labeled anti-CD9 antibody, an HRP-labeled anti-CD63 antibody, and an HRP-labeled anti-CD81 antibody were diluted by 500-fold respectively with a dilution buffer, was added to each well and incubated at room temperature. After 2 hours, the plate was washed 5 times with a washing solution, a TMB substrate was added, and when color developed, a reaction stop solution was added, and the absorbance at 450 nm was immediately measured with a microplate reader. The absorbance in the figure is the value obtained by subtracting the blank value from each value. The results are shown in FIG. 34.

### Comparative Example 25

### Detection of extracellular vesicles derived from a normal mammary epithelial cell line by Tim4 polypeptide

Extracellular vesicles were detected using HMEC-TKD cells, which are a normal mammary epithelial cell line, instead of the breast cancer cell lines in Comparative Example 24 above. The results are shown in FIG. 34.

The results of Example 15 and Comparative Examples 23 to 25 above show that purified extracellular vesicles derived from MDA-MB-231 cells, MDA-MB-45 cells, and T47D cells are captured by the VAR2CSA polypeptide and Tim4 and detected by the cocktail antibody of the anti-CD9 antibody, anti-CD63 antibody, and anti-CD81 antibody, whereas purified extracellular vesicles derived from HMEC-TKD are captured by Tim4 and detected by the cocktail antibody of the anti-CD9 antibody, anti-CD63 antibody, and anti-CD81 antibody, but are not captured by the VAR2CSA polypeptide.

### Example 16

### Detection of extracellular vesicles derived from an ovarian cancer cell line by VAR2CSA polypeptide

Extracellular vesicles prepared from the ovarian cancer cell line OVCAR3 cells by the method described in Reference Example 6 were diluted, and captured and detected by the method described above in section A. Specifically, the purified EVs sample was diluted by 2.5-fold and added to each well of a VAR2CSA-immobilized plate. The VAR2CSA-immobilized plate was prepared by adding the VAR2CSA polypeptide to a nickel chelate-coated plate. After incubation overnight at 4°C, and washing 5 times with PBST, an antibody cocktail solution, in which an HRP-labeled anti-CD9 antibody, an HRP-labeled anti-CD63 antibody, and an HRP-labeled anti-CD81 antibody were diluted by 500-fold respectively with a dilution buffer, was added to each well and incubated at room temperature. After 2 hours, the plate was washed 5 times with PBST, a TMB substrate was added, and when color developed, a reaction stop solution was added, and the absorbance at 450 nm was immediately measured with a microplate reader. The absorbance in the figure is the value obtained by subtracting the blank value from each value. The results are shown in FIG. 35.

### Example 17

Detection of extracellular vesicles derived from a bladder cancer cell line by VAR2CSA polypeptide

Extracellular vesicles were detected using the bladder cancer cell line T24 cells instead of the ovarian cancer cell line in Example 16 above. The results are shown in FIG. 35.

### Comparative Example 26

### Detection of extracellular vesicles derived from an ovarian cancer cell line by Tim4 polypeptide

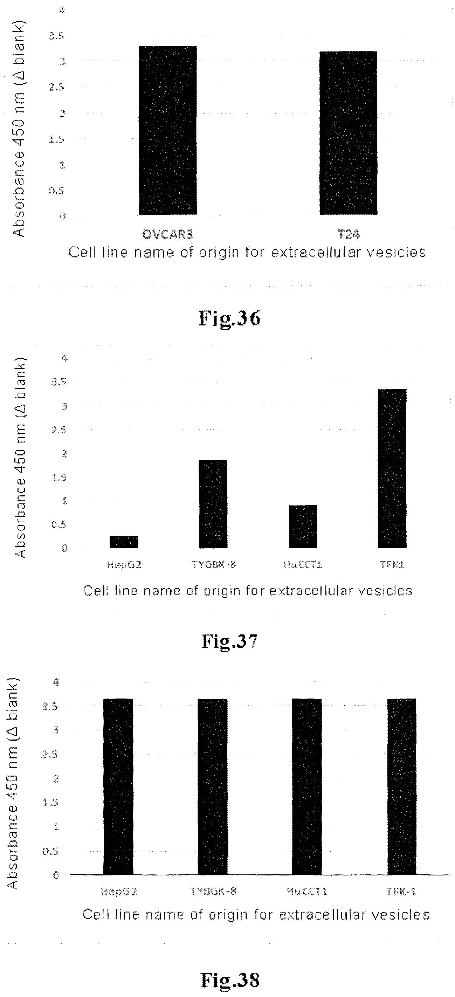

In addition, purified extracellular vesicle samples derived from OVCAR3 cells diluted by 25-fold were added to each well of a Tim4-immobilized plate. After incubation overnight at 4°C, and washing 5 times with a washing solution, an antibody cocktail solution, in which an HRP-labeled anti-CD9 antibody, an HRP-labeled anti-CD63 antibody, and an HRP-labeled anti-CD81 antibody were diluted by 500-fold respectively with a dilution buffer, was added to each well and incubated at room temperature. After 2 hours, the plate was washed 5 times with a washing solution, a TMB substrate was added, and when color developed, a reaction stop solution was added, and the absorbance at 450 nm was immediately measured with a microplate reader. The absorbance in the figure is the value obtained by subtracting the blank value from each value. The results are shown in FIG. 36.

### Comparative Example 27

### Detection of extracellular vesicles derived from a bladder cancer cell line by Tim4 polypeptide

Extracellular vesicles were detected using the bladder cancer cell line T24 cells instead of the ovarian cancer cell line in Comparative Example 26 above. The results are shown in FIG. 36.

The results of Examples 16 and 17 and Comparative Examples 26 and 27 above show that purified extracellular vesicles derived from the ovarian cancer cell line OVCAR3 cells and the bladder cancer cell line T24 cells are captured by the VAR2CSA polypeptide and Tim4 and detected by the cocktail antibody of the anti-CD9 antibody, anti-CD63 antibody, and anti-CD81 antibody.

### Examples 18 to 20

### Detection of extracellular vesicles derived from a liver cancer cell line, a gallbladder cancer cell line, and bile duct cancer cell lines by VAR2CSA polypeptide

Extracellular vesicles prepared by the method described in Reference Example 6 from the liver cancer cell line HepG2 cells (Example 18), the gallbladder cancer cell line TYGBK-8 cells (Example 19), and the bile duct cancer cell lines HuCCT1 cells and TFK-1 cells (all Example 20) were diluted, and captured and detected by the method described above in section A. Specifically, the purified EVs sample was diluted by 2.5-fold and added to each well of a VAR2CSA-immobilized plate. The VAR2CSA-immobilized plate was prepared by adding the VAR2CSA polypeptide to a nickel chelate-coated plate. After incubation overnight at 4°C, and washing 5 times with PBST, an antibody cocktail solution, in which an HRP-labeled anti-CD9 antibody, an HRP-labeled anti-CD63 antibody, and an HRP-labeled anti-CD81 antibody were diluted by 500-fold respectively with a dilution buffer, was added to each well and incubated at room temperature. After 2 hours, the plate was washed 5 times with PBST, a TMB substrate was added, and when color developed, a reaction stop solution was added, and the absorbance at 450 nm was immediately measured with a microplate reader. The absorbance in the figure is the value obtained by subtracting the blank value from each value. The results are shown in FIG. 37.

Comparative Examples 28 to 30

### Detection of extracellular vesicles derived from a liver cancer cell line, a gallbladder cancer cell line, and bile duct cancer cell lines by Tim4 polypeptide

In addition, purified extracellular vesicle samples derived from HepG2 cells (Comparative Example 28), TYGBK-8 cells (Comparative Example 29), and HuCCT1 cells and TFK-1 cells (all Comparative Example 30) diluted by 10-fold were added to each well of a Tim4-immobilized plate. After incubation overnight at 4°C, and washing 5 times with a washing solution, an antibody cocktail solution, in which an HRP-labeled anti-CD9 antibody, an HRP-labeled anti-CD63 antibody, and an HRP-labeled anti-CD81 antibody were diluted by 500-fold respectively with a dilution buffer, was added to each well and incubated at room temperature. After 2 hours, the plate was washed 5 times with a washing solution, a TMB substrate was added, and when color developed, a reaction stop solution was added, and the absorbance at 450 nm was immediately measured with a microplate reader. The absorbance in the figure is the value obtained by subtracting the blank value from each value. The results are shown in FIG. 38.

The results of Examples 18 to 20 and Comparative Examples 28 to 30 above show that purified extracellular vesicles derived from the liver cancer cell line HepG2 cells, the gallbladder cancer cell line TYGBK-8 cells, and the bile duct cancer cell lines HuCCT1 cells and TFK-1 cells are captured by the VAR2CSA polypeptide and Tim4 and detected by the cocktail antibody of the anti-CD9 antibody, anti-CD63 antibody, and anti-CD81 antibody.

### Example 21

### Detection of extracellular vesicles derived from an acute lymphocytic leukemia cell line by VAR2CSA polypeptide

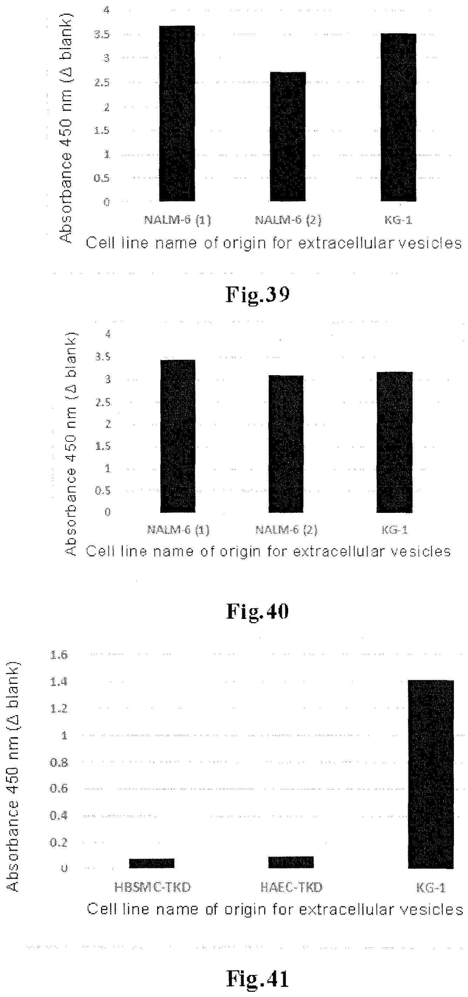

Extracellular vesicles prepared from the acute lymphocytic leukemia cell line NALM-6 cells by the method described in Reference Example 6 were diluted, and captured and detected by the method described above in section A. For the preparation of EVs from NALM-6 cells, samples (1) and (2) prepared in two independent experiments were used. Specifically, these purified EVs samples were diluted by 2.5-fold and added to each well of a VAR2CSA-immobilized plate. The VAR2CSA-immobilized plate was prepared by adding the VAR2CSA polypeptide to a nickel chelate-coated plate. After incubation overnight at 4°C, and washing 5 times with PBST, an antibody cocktail solution, in which an HRP-labeled anti-CD9 antibody, an HRP-labeled anti-CD63 antibody, and an HRP-labeled anti-CD81 antibody were diluted by 500-fold respectively with a dilution buffer, was added to each well and incubated at room temperature. After 2 hours, the plate was washed 5 times with PBST, a TMB substrate was added, and when color developed, a reaction stop solution was added, and the absorbance at 450 nm was immediately measured with a microplate reader. The absorbance in the figure is the value obtained by subtracting the blank value from each value. The results are shown in FIG. 39.

### Example 22

### Detection of acute myeloid leukemia cell line-derived extracellular vesicles by VAR2CSA polypeptide

An attempt was made to detect extracellular vesicles using acute myeloid leukemia cell line KG-1 cells instead of the acute lymphocytic leukemia cell line in Example 21 above. The results are shown in FIG. 39.

### Comparative Example 31

### Detection of acute lymphocytic leukemia cell line-derived extracellular vesicles by Tim4 polypeptide

Furthermore, 25-fold diluted NALM-6 cell-derived purified extracellular vesicle samples were added to each well of a Tim4-immobilized plate. After incubation overnight at 4°C, the plate was washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The absorbance in the figure is the value obtained by subtracting the blank value from each value. The results are shown in FIG. 40.

### Comparative Example 32

### Detection of acute myeloid leukemia cell line-derived extracellular vesicles by Tim4 polypeptide

An attempt was made to detect extracellular vesicles using KG-1 cells instead of the NALM-6 cells in Comparative Example 30 above. The results are shown in FIG. 40.

The results of Examples 21 and 22 and Comparative Examples 31 and 32 above show that NALM-6 cell- and KG-1 cell-derived purified extracellular vesicles are captured by the VAR2CSA polypeptide and Tim4 and detected by the cocktail antibody of anti-CD9 antibody, anti-CD63 antibody, and anti-CD81 antibody.

### Comparative Example 33

### Attempt of detection of extracellular vesicles derived from normal tracheal smooth muscle cell line by VAR2CSA polypeptide

Extracellular vesicles prepared by the method described in Reference Example 6 from the tracheal smooth muscle cell line HBSMC-TKD cells were diluted, and captured and detected by the method described above in section A. Specifically, the purified EVs sample was diluted by 2.5-fold and added to each well of the VAR2CSA-immobilized plate. The VAR2CSA-immobilized plate was prepared by adding the VAR2CSA polypeptide to a nickel chelate-coated plate. After incubation overnight at 4°C, the plate was washed 5 times with PBST, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The absorbance in the figure is the value obtained by subtracting the blank value from each value. The results are shown in FIG. 41.

### Comparative Example 34

### Attempt of detection of extracellular vesicles derived from normal aortic endothelial cell line by VAR2CSA polypeptide

An attempt was made to detect extracellular vesicles using aortic endothelial cell line HAEC-TKD cells instead of the tracheal smooth muscle cell line HBSMC-TKD cells in Comparative Example 33 above. The results are shown in FIG. 41.

### Example 23

### Detection of acute myeloid leukemia cell line-derived extracellular vesicles by VAR2CSA polypeptide

Extracellular vesicles were detected using acute myeloid leukemia cell line KG-1 cells instead of the tracheal smooth muscle cell line HBSMC-TKD cells in Comparative Example 32 above. The results are shown in FIG. 41.

### Comparative Example 35

Detection of extracellular vesicles derived from normal tracheal smooth muscle cell line by Tim4 polypeptide

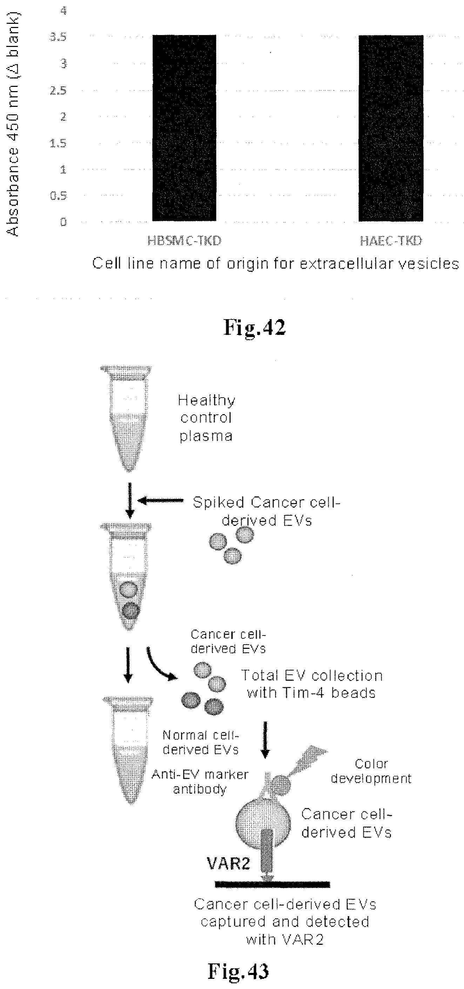

Furthermore, 10-fold diluted HBSMC-TKD cell-derived purified extracellular vesicle samples were added to each well of a Tim4-immobilized plate. After incubation overnight at 4°C, the plate was washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The absorbance is the value obtained by subtracting the blank value from each value. The results are shown in FIG. 42.

### Comparative Example 36

### Detection of extracellular vesicles derived from normal aortic endothelial cell line by Tim4 polypeptide

Extracellular vesicles were detected using HAEC-TKD cells instead of the HBSMC-TKD cells in Comparative Example 34 above. The results are shown in FIG. 42.

The results of Example 23 and Comparative Examples 33 to 36 above show that purified extracellular vesicles derived from normal cells HBSMC-TKD cells and HAEC-TKD cells are not captured by the VAR2CSA polypeptide, but are captured by Tim4 and detected by the cocktail antibody of anti-CD9 antibody, anti-CD63 antibody, and anti-CD81 antibody.

Furthermore, the results of Examples 14 to 23 and Comparative Examples 20 to 36 are summarized in Table 8. In the table, O indicates that it was captured, and × indicates that it was not captured. It is shown that extracellular vesicles derived from kidney cancer cells (Caki-1 cells, Caki-2 cells), breast cancer cells (MDA-MB-231 cells, MDA-MB-453 cells, T47D cells), ovarian cancer cells (OVCAR3 cells), bladder cancer cells (T24 cells), liver cancer cells (HepG2 cells), gallbladder cancer cells (TYGBK-8 cells), bile duct cancer cells (HuCCT1 cells, TFK-1 cells), acute lymphocytic leukemia cell line (NALM-6 cells), and acute myeloid leukemia cell line (KG-1 cells) are captured, but extracellular vesicles derived from normal cells, kidney epithelial cells (HREC-TKD cells), breast epithelial cells (HMEC-TDK cells), tracheal smooth muscle cells (HBSMC-TKD cells), and aortic endothelial cells (HAEC-TKD cells) are not captured.

**[Table 8]**

| Site | Cancer type/normal cell | Cell name | Capturing molecule VAR2 | Capturing molecule Tim4 |
|---|---|---|---|---|
| kidney | kidney cancer | Caki-1 | ○ | ○ |
| | | Caki-2 | ○ | ○ |
| | kidney epithelial cell | HREC-TKD | × | ○ |
| breast | breast cancer | MDA-MB-231 | ○ | ○ |
| | | MDA-MB-453 | ○ | ○ |
| | | T47D | ○ | ○ |
| | breast epithelial cell | HMEC-TDK | × | ○ |
| ovary | ovary cancer | OVCAR3 | ○ | ○ |
| bladder | bladder cancer | T24 | ○ | ○ |
| liver | liver cancer | HepG2 | ○ | ○ |
| gallbladder | gallbladder cancer | TYGBK-8 | ○ | ○ |
| bile duct | bile duct cancer | HuCCT1 | ○ | ○ |
| | | TFK-1 | ○ | ○ |
| blood | acute lymphocytic leukemia | NALM-6 | ○ | ○ |
| blood | acute myeloid leukemia | KG-1 | ○ | O |
| bronchi | tracheal smooth muscle cell | BSMC-TKD | × | O |
| blood vessel | aortic endothelial cell | HAEC-TKD | × | ○ |

### Example 24

Detection by VAR2CSA polypeptide of acute myeloid leukemia cell line KG-1 cell-derived extracellular vesicles added to human healthy control plasma

EVs were purified from a sample of 1 mL of healthy control plasma spiked with 25 µL of EVs purified from KG-1 cell culture supernatant, using MagCapture (tradename) Exosome Isolation Kit PS Ver. 2 (manufactured by Fujifilm). A scheme of the procedure is shown in FIG. 43. Furthermore, immobilization of the VAR2 polypeptide to the plate was performed by adding the VAR2 polypeptide derived from the 7G8 strain to a nickel chelate-coated plate (Thermo Scientific).

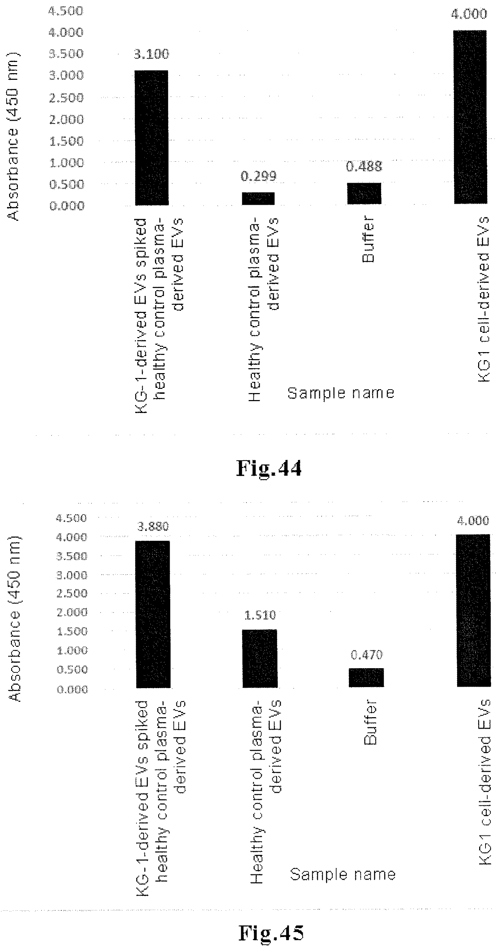

90 µL out of 100 µL of EVs purified from the plasma spiked with EVs was added to the 7G8 strain VAR2 polypeptide-immobilized plate. The added sample volume was adjusted to 100 µL by adding buffer. After incubation for 18 hours at 4°C and washing with washing buffer, a mixed antibody solution of HRP-anti-CD9 antibody, HRP-anti-CD63 antibody, and HRP-anti-CD81 antibody, diluted with antibody dilution buffer, was added and incubated for 2 hours at room temperature. After washing, TMB solution, which is a peroxidase color-developing substrate, was added and reacted for 10 minutes. Color development stop solution was added thereto to stop the reaction, and the absorbance at 450 nm of each well was immediately measured. The results are shown in FIG. 44. Cancer-derived EVs spiked into healthy control plasma were captured by VAR2CSA, similar to the 25 µL of KG-1 cell-derived purified EVs used for spiking.

### Comparative Example 37

### Attempt of detection by VAR2CSA polypeptide of extracellular vesicles derived from human healthy control plasma

In Comparative Example 35, an attempt was made to detect extracellular vesicles using 90 µL of EVs purified from the same control plasma not spiked with EVs, instead of the plasma spiked with EVs. The results are shown in FIG. 44.

### Comparative Example 38

### Detection by Tim4 polypeptide of acute myeloid leukemia cell line KG-1 cell-derived extracellular vesicles added to human healthy control plasma

Next, it was confirmed whether these extracellular vesicles could be captured by Tim4. Briefly, 10 µL of the prepared EVs was adjusted to 100 µL with buffer and added to a Tim4 polypeptide-immobilized plate. At the same time, 10 µL of the KG-1 cell-derived purified EVs used for spiking was also adjusted to 100 µL with buffer and added to a Tim4 polypeptide-immobilized plate. After overnight incubation at 4°C, the plate was washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 45. Tim4 was able to capture the extracellular vesicles of all samples.

### Comparative Example 39

### Detection by Tim4 polypeptide of extracellular vesicles derived from human healthy control plasma

In Comparative Example 37, extracellular vesicles were detected using the same control plasma not spiked with EVs, instead of the plasma spiked with EVs. The results are shown in FIG. 45.

### Example 25

### Detection of extracellular vesicles in colon cancer patient plasma by VAR2CSA polypeptide

It was investigated whether cancer cell-derived extracellular vesicles in colon cancer patient plasma can be captured by the VAR2CSA polypeptide. Immobilization of the VAR2 polypeptide to the plate was performed by adding the VAR2 polypeptide derived from the 7G8 strain to a nickel chelate-coated plate (Thermo Scientific).

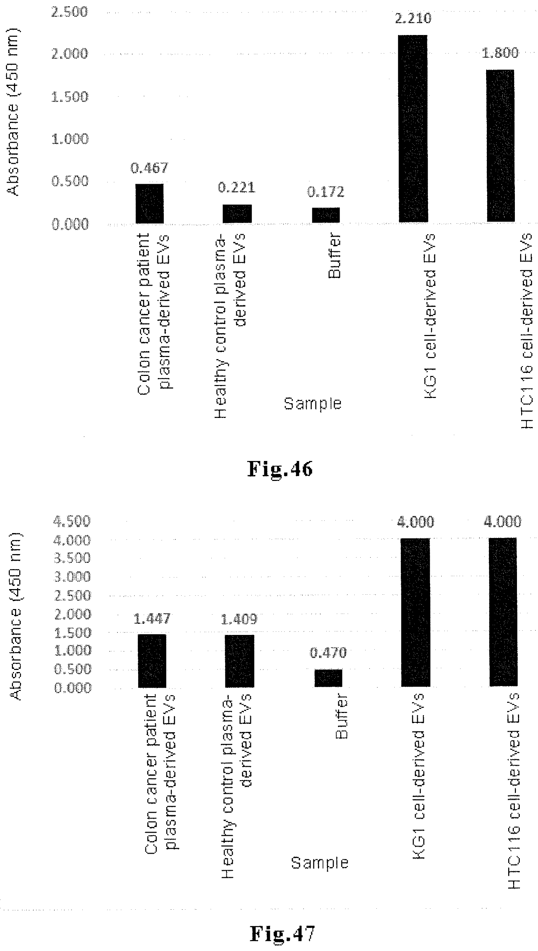

EVs were purified from 1 mL each of colon cancer patient plasma (purchased from National BioService, Stage IV) and healthy control plasma (purchased from National BioService) using MagCapture (tradename) Exosome Isolation Kit PS Ver. 2 (manufactured by Fujifilm). 90 µL out of 100 µL of purified EVs was adjusted to 100 µL with buffer and added to the 7G8 strain VAR2 polypeptide-immobilized plate. As controls, 100 µL of buffer, EVs purified from leukemia cell line KG-1 cell culture supernatant, and EVs purified from colon cancer cell line HTC-116 cell culture supernatant, diluted by 2.5-fold with buffer, were added to the 7G8 strain VAR2 polypeptide-immobilized plate. After incubation for 18 hours at 4°C, the plate was washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 46.

### Comparative Example 40

### Detection of extracellular vesicles in colon cancer patient plasma by Tim4 polypeptide

Next, samples prepared by adding buffer to 10 µL of each purified EVs to make 100 µL were added to a Tim4-immobilized plate. After incubation for 18 hours at 4°C, the plate was washed 5 times with washing solution, and an antibody cocktail solution, in which HRP-labeled anti-CD9 antibody, HRP-labeled anti-CD63 antibody, and HRP-labeled anti-CD81 antibody were diluted by 500-fold respectively with dilution buffer, was added to each well and incubated at room temperature. After 2 hours, the plate was washed 5 times with PBST, TMB substrate was added, and when color developed, stop solution was added, and the absorbance at 450 nm was immediately measured with a microplate reader. The results are shown in FIG. 47.

According to Examples 24 and 25 and Comparative Examples 37 to 40, extracellular vesicles purified from colon cancer patient plasma were captured by VAR2, but extracellular vesicles from healthy control plasma were not captured by VAR2. On the other hand, Tim4 captured all extracellular vesicles.

From the above Examples, it was shown that extracellular vesicles derived from glioblastoma cells (U87-MG cells), pancreatic cancer cells (KMP2 cells, PANC-1 cells, MIA Paca-2 cells), prostate cancer cells (PC-3 cells, LNCap cells), lung cancer cells (NCI-H1703 cells, H69AR cells, A549 cells), osteosarcoma (MG-63 cells), esophageal cancer cells (KYSE150 cells, KYSE510 cells, KYSE1240 cells), stomach cancer cells (HGC-27 cells, AGS cells, MKN45 cells), colon cancer cell lines (SW837 cells, SW480 cells, HTC-116 cells, Caco-2 cells), kidney cancer cells (Caki-1 cells, Caki-2 cells), breast cancer cells (MDA-MB-231 cells, MDA-MB-453 cells, T47D cells), ovarian cancer cells (OVCAR3 cells), bladder cancer cells (T24 cells), liver cancer cells (HepG2 cells), gallbladder cancer cells (TYGBK-8 cells), bile duct cancer cells (HuCCT1 cells, TFK-1 cells), acute myeloid leukemia cells (KG-1 cells), and acute lymphocytic leukemia cells (NALM-6 cells) can be captured and detected by the method of the present invention. Furthermore, it was shown that extracellular vesicles derived from biological samples of patients having cancer can be captured and detected by the method of the present invention. Thus, according to the method of the present invention, since it is possible to detect extracellular vesicles derived from a wide range of cancer cells belonging to different categories, such as epithelial cancer, sarcoma, and blood cancer, it is reasonably considered that cancer can be detected regardless of the type of cancer.

### INDUSTRIAL APPLICABILITY

The method of isolating cancer-derived extracellular vesicles of the present invention provides a method of capturing and detecting cancer cell-derived extracellular vesicles in a biological sample and a kit, and can be used for cancer diagnosis.

## Claims

1. A method of capturing cancer cell-derived extracellular vesicles contained in a biological sample, the method comprising a step of contacting the biological sample with any one polypeptide of (A) to (C) below to bind the polypeptide and the cancer cell-derived extracellular vesicles:
(A) a polypeptide containing ID1-ID2a domains of VAR2CSA protein derived from *Plasmodium falciparum*;
(B) a polypeptide having 90% or more sequence identity with amino acid sequence of the polypeptide of (A) above, and binding to cancer cell-derived extracellular vesicles;
(C) a part of the polypeptide of (A) or (B) above, which is a polypeptide binding to cancer cell-derived extracellular vesicles.

2. The method of claim 1, wherein the ID1-ID2a domains of the VAR2CSA protein has an amino acid sequence of SEQ ID NO: 1, SEQ ID NO: 9, SEQ ID NO: 12, SEQ ID NO: 13, or SEQ ID NO: 14.

3. The method of claim 1 or 2, wherein the polypeptide of (A) to (C) is immobilized on a support.

4. The method of claim 1 or 2, wherein the biological sample is cell culture supernatant, blood, serum, or plasma of a human.

5. A method of detecting cancer cell-derived extracellular vesicles contained in a biological sample, the method comprising:
a step of contacting the biological sample with any one polypeptide of (A) to (C) below to bind the polypeptide and the cancer cell-derived extracellular vesicles; and
a step of detecting the cancer cell-derived extracellular vesicles bound to any one polypeptide of (A) to (C):
(A) a polypeptide containing ID1-ID2a domains of VAR2CSA protein derived from *Plasmodium falciparum*;
(B) a polypeptide having 90% or more sequence identity with amino acid sequence of the polypeptide of (A) above, and binding to cancer cell-derived extracellular vesicles;
(C) a part of the polypeptide of (A) or (B) above, which is a polypeptide binding to cancer cell-derived extracellular vesicles.

6. The method of claim 5, further comprising contacting the cancer cell-derived extracellular vesicles with a second binding substance that binds to a surface of the extracellular vesicles, and comprising detecting the cancer cell-derived extracellular vesicles bound to the second binding substance and any one polypeptide of (A) to (C).

7. The method of claim 6, wherein:
either one of (i) any one polypeptide of (A) to (C) and (ii) the second binding substance is immobilized on a support, and the other is labeled; and
the detection is performed by detecting the label.

8. The method of claim 7, wherein the second binding substance is an antibody or an antigen-binding fragment thereof, or a phosphatidylserine-binding domain of Tim1, Tim3, Tim4, or beta-2-glycoprotein 1 (B2GP1), which is a protein that binds to phosphatidylserine located on outer side of membrane of the extracellular vesicles.

9. The method of claim 8, wherein the antibody is an antibody against CD9, CD63, or CD81.

10. The method of claim 6, wherein the biological sample is cell culture supernatant, blood, serum, or plasma of a human.

11. The method of any one of claims 5 to 10, wherein the ID1-ID2a domains of the VAR2CSA protein has an amino acid sequence of SEQ ID NO: 1, SEQ ID NO: 9, SEQ ID NO: 12, SEQ ID NO: 13, or SEQ ID NO: 14.

12. A method of obtaining information on whether or not a subject has cancer, the method being performed by detecting cancer cell-derived extracellular vesicles contained in a biological sample obtained from the subject by the method of any one of claims 1 to 11.

13. A kit comprising:
any one polypeptide of (A) to (C) below; and
a second binding substance that binds to a surface of extracellular vesicles,
wherein either one of (i) any one polypeptide of (A) to (C) and (ii) the second binding substance is immobilized on a support, and the other is labeled:
(A) a polypeptide comprising ID1-ID2a domains of VAR2CSA protein derived from *Plasmodium falciparum*;
(B) a polypeptide having 90% or more sequence identity with amino acid sequence of the polypeptide of (A) above, and binding to cancer cell-derived extracellular vesicles;
(C) a part of the polypeptide of (A) or (B) above, which is a polypeptide binding to cancer cell-derived extracellular vesicles.

14. The kit of claim 13, wherein the kit is for diagnosing whether or not a subject has cancer.
